(19) Europäisches Patentamt
European Patent Office
Office européen des brevets

(11) Veröffentlichungsnummer: **0 332 009 B1**

# (12) EUROPÄISCHE PATENTSCHRIFT

(45) Veröffentlichungstag der Patentschrift: **21.04.93**

(51) Int. Cl.[5]: **C07D 413/14**, C07D 413/04, C07D 417/14, C07D 417/04, C07D 405/14, C07D 403/04, C07D 409/14, A01N 43/34, A01N 43/76, A01N 43/78, A01N 43/60

(21) Anmeldenummer: **89103439.9**

(22) Anmeldetag: **28.02.89**

Die Akte enthält technische Angaben, die nach dem Eingang der Anmeldung eingereicht wurden und die nicht in dieser Patentschrift enthalten sind.

## (54) N-Aryltetrahydrophthalimidverbindungen.

(30) Priorität: **05.03.88 DE 3807295**

(43) Veröffentlichungstag der Anmeldung:
**13.09.89 Patentblatt 89/37**

(45) Bekanntmachung des Hinweises auf die Patenterteilung:
**21.04.93 Patentblatt 93/16**

(84) Benannte Vertragsstaaten:
**BE CH DE ES FR GB IT LI NL**

(56) Entgegenhaltungen:
**EP-A- 0 170 191**
**EP-A- 0 177 032**
**EP-A- 0 218 972**
**EP-A- 0 263 299**

(73) Patentinhaber: **BASF Aktiengesellschaft**
**Carl-Bosch-Strasse 38**
**W-6700 Ludwigshafen(DE)**

(72) Erfinder: **Rueb, Lothar, Dr.**
**Zum Weidentor 4**
**W-6720 Speyer(DE)**
Erfinder: **Eicken, Karl, Dr.**
**Am Huettenwingert 12**
**W-6706 Wachenheim(DE)**
Erfinder: **Plath, Peter, Dr.**
**Hans-Balcke-Strasse 13**
**W-6710 Frankenthal(DE)**
Erfinder: **Schwalge, Barbara, Dr.**
**Adolf-Diesterweg-Strasse 77**
**W-6700 Ludwigshafen(DE)**
Erfinder: **Westphalen, Karl-Otto, Dr.**
**Mausbergweg 58**
**W-6720 Speyer(DE)**
Erfinder: **Wuerzer, Bruno, Dr.**
**Ruedigerstrasse 13**
**W-6701 Otterstadt(DE)**
Erfinder: **Meyer, Norbert, Dr.**
**Dossenheimer Weg 22**
**W-6802 Ladenburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 332 009 B1

## Beschreibung

Die vorliegende Erfindung betrifft N-Phenyltetrahydrophthalimidverbindungen der allgemeinen Formel I,

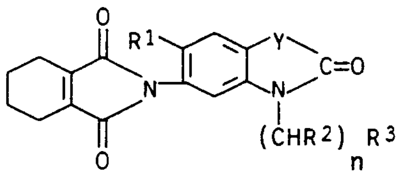

I

in der die Substituenten und Indices folgende Bedeutung haben:

n die Zahl 0 oder 1

$R^1$ Wasserstoff oder Halogen

Y -O-, -S-, $-CH_2-$
$-S-CR^4R^5-$,
$-O-CR^4R^5-$,
$-CH_2-CR^4R^5-$,

wobei $R^4$ und $R^5$ unabhängig voneinander Wasserstoff oder $C_1$-$C_3$-Alkyl bedeuten,

$R^2$ Wasserstoff oder $C_1$-$C_3$-Alkyl

$R^3$ einen gesättigten oder einfach ungesättigten Fünf- oder Sechsring mit einem Sauerstoff- oder Schwefelatom im Ring, der durch $C_1$-$C_3$-Alkyl ein- bis dreifach substituiert sein kann oder unsubstituiertes oder ein- bis dreifach durch $C_1$-$C_2$-Alkyl, Halogen, Methoxy, Nitro und/oder Cyano substituiertes Phenyl und
sofern n = 0 ist und Y die Bedeutung $-CH_2-$, $-CH_2-CR^4R^5-$ oder $-S-CR^4R^5-$ hat,
$R^3$ zusätzlich Wasserstoff, $C_1$-$C_5$-Alkyl, $C_3$-$C_4$-Alkenyl, $C_3$-$C_4$-Alkinyl, $C_1$-$C_4$-Haloalkyl, $C_3$-$C_4$-Haloalkenyl, $C_1$-$C_2$-Alkoxy-($C_1$-$C_2$)-alkyl, $C_1$-$C_2$-Alkoxy-($C_1$-$C_2$)-alkoxy-($C_1$-$C_2$)-alkyl oder durch $C_1$-$C_6$-Alkoxycarbonyl substituiertes $C_1$-$C_3$-Alkyl.

Weiterhin betrifft die Erfindung Verfahren zur Herstellung der Verbindungen I, deren Verwendung zur Bekämpfung unerwünschten Pflanzenwuchses sowie herbizide Mittel, die diese Verbindungen enthalten.

Es sind bereits N-arylsubstituierte Tetrahydrophthalimide mit Herbizidwirkung bekannt. So werden z.B. in EP-A-170 191 Tetrahydrophthalimide beschrieben, deren Wirksamkeit bei niedrigen Aufwandmengen allerdings unbefriedigend ist.

Ferner beschreibt die EP-A-177 032 herbizid wirksame Tetrahydrophthalimide der Formel I'

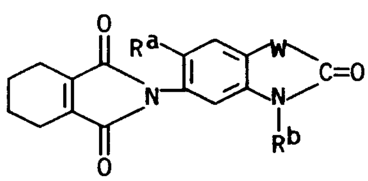

I'

wobei
$R^a$ Wasserstoff, Fluor oder Chlor, w eine Gruppe $-CH(R^c)-N(R^d)-$ oder $-C(R^c)=N-$ (mit $R^c$, $R^d$ = Wasserstoff oder Methyl) und $R^b$ eine Alkyl-, Alkenyl-, Alkinyl- oder Alkoxymethylgruppe bedeuten.

Außerdem sind der EP-A-218 972 Benzothiazolone der Formel II'

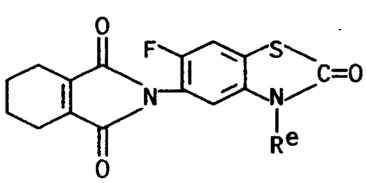

I''

mit ebenfalls herbizider Aktivität zu entnehmen, wobei $R^e$ für Wasserstoff, Alkyl, Alkenyl, Alkinyl, Halogenalkyl, Halogenalkenyl, Halogenalkinyl, Alkoxyalkyl, Alkoxyalkoxyalkyl, Cinnamyl, Cyanoalkyl, Carboxyalkyl,

Alkoxycarbonylalkyl, Halogenalkoxycarbonylalkyl, Alkoxyalkoxycarbonylalkyl, Alkoxycarbonylalkoxycarbonylalkyl, Cycloalkoxycarbonylalkyl, Alkylaminocarbonylalkyl oder Dialkylaminocarbonylalkyl steht.

Die herbizide Wirkung dieser bekannten Verbindungen bezüglich Aufwandmenge und Selektivität vermag jedoch nur bedingt zu befriedigen.

Der Erfindung lag die Aufgabe zugrunde, verbesserte Tetrahydrophthalimidverbindungen zu finden und zu synthetisieren, mit denen sich die Schadpflanzen besser als bisher gezielt bekämpfen lassen.

Es wurde gefunden, daß die eingangs definierten N-Arylhtetrahydrophthalimidverbindungen der Formel I eine vorteilhafte herbizide Wirkung, besonders im Nachauflaufverfahren haben und gegenüber einer Reihe von Kulturpflanzen selektiv sind. Soweit diese Verbindungen Stereoisomere bilden können, bezieht sich die Erfindung auf alle Isomere.

Man erhält die Verbindungen I beispielsweise, indem man ein entsprechend substituiertes Anilin der Formel II zuerst in an sich bekannter Weise in einem inerten organischen Lösungsmittel mit einer N-alkylierenden Verbindung Z-$(CHR^2)_nR^3$ zum Anilinderivat III umsetzt, und dieses anschließend in einem inerten organischen Lösungsmittel mit 3,4,5,6-Tetrahydrophthalsäureanhydrid zum N-Aryltetrahydrophthalimid I kondensiert

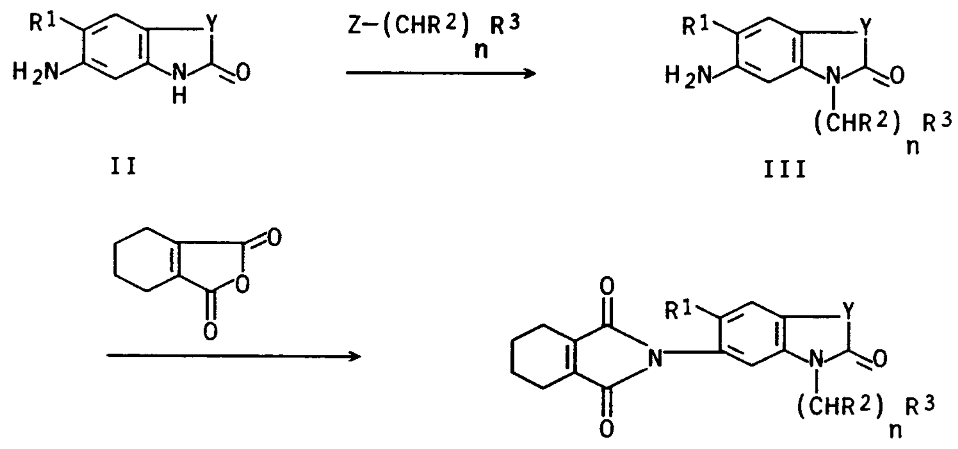


II

III

I

Der Rest Z im Alkylierungsreagens bedeutet eine in der organischen Chemie übliche nucleophile Abgangsgruppe wie Halogen z.B. Chlor, Brom und Iod oder Sulfonylrest wie Toluolsulfonyl- und Mesylrest.

Die Alkylierung erfolgt in an sich bekannter Weise bei bis zu 200 ° C, vorzugsweise bei 0 bis 150 ° C, in einem aprotisch polaren Lösungsmittel (z.B. Aceton, Acetonitril, Dimethylformamid), in Gegenwart einer Base (z.B. Kaliumcarbonat, Natriumhydroxid, Natriumhydrid).

Die Kondensation des Anilinderivates III mit 3,4,5,6-Tetrahydrophthalsäureanhydridwird in protischen Lösungsmitteln wie Essigsäure, Propionsäure und iso-Buttersäure oder in aprotischen LÖsungsmitteln wie Toluol, Xylol und Dimethylformamid durchgeführt. Die Umsetzung erfolgt in der Regel bei Temperaturen von 25 ° C bis 200 ° C, vorzugsweise bei 70 ° C bis 140 ° C. Beim Arbeiten in aprotischen Lösungsmitteln empfiehlt es sich, das Wasser fortlaufend zu entfernen.

Man erhält die Verbindungen I aber auch, wenn man das Amin II zunächst mit 3,4,5,6-Tetrahydrophthalsäureanhydrid zum N-(Phenyl)tetrahydrophthalimid IV kondensiert und dieses anschließend mit einer N-alkylierenden Verbindung Z-$(CHR^2)_nR^3$ zu I umsetzt.

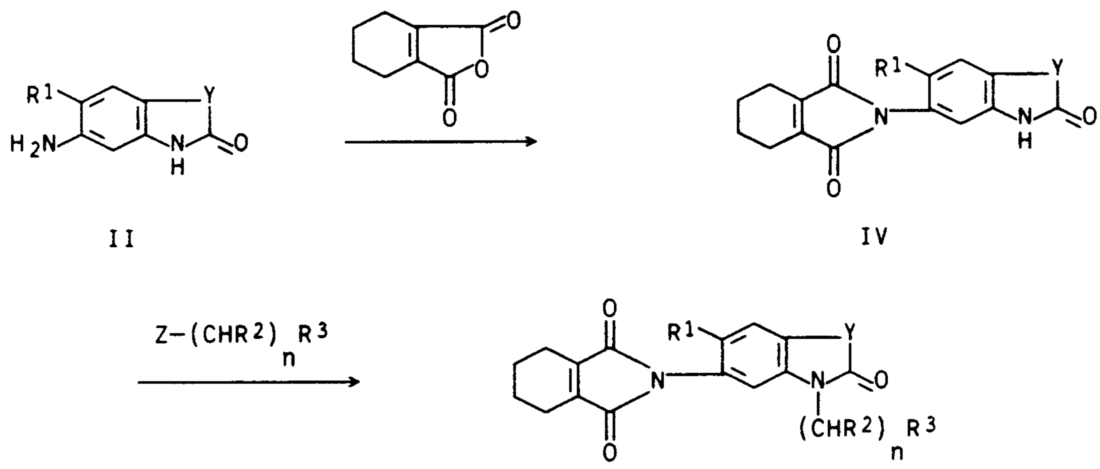


II IV I

Diese Umsetzungen erfolgen analog den vorstehend geschilderten Bedingungen.

Die Darstellung der benötigten Verbindungen II erfolgt auf an sich bekannte Weise: S. Gabriel et al., Chem. Ber. 12 (1879) 600; W.A. Sexton, J. Chem. Soc. 1939, 470; D.R. Shridar et al. Synthesis 1982, 986; Sandmeyer, Chem. Ber. 19 (1886) 2656; D.R. Shridar et al. Ind. J. Chem. 24B (1985) 1263.

Im Hinblick auf die bestimmungsgemäße Verwendung der Verbindungen I kommen als Substituenten bevorzugt die folgenden Reste in Betracht.

Unter Halogen wie vorstehend verwendet, ist Fluor, Chlor oder Brom zu verstehen, wobei $R^1$ in der Bedeutung für Halogen bevorzugt für Fluor steht.

Die Bezeichnung Alkyl umfaßt verzweigte und geradkettige Reste, z.B. Methyl, Ethyl, n-Propyl, Isopropyl.

Die Alkenyl- und Alkinylreste können ebenfalls verzweigt oder geradkettig sein. Beispiele für Alkenylreste in Formel I sind Allyl, 2-Propenyl, 2-Butenyl, 3-Butenyl, 2-Isobutenyl, insbesondere Allyl oder 2-Butenyl, Alkinylreste sind in der Regel Propargyl, 2-Butinyl, 3-Butinyl.

Für den heterocyclischen Fünf- oder Sechsring der Formel I steht bevorzugt ein hydrierter oder teilhydrierter Furan-, Thiophen-, Pyran- und Thiopyran-Ring.

Die Bezeichnung Phenyl umfaßt bevorzugt die unsubstituierten und monosubstituierten Derivate, z.B. Chlorphenyl, Methylphenyl, Methoxyphenyl.

Die N-substituierten Tetrahydrophthalimide sind aus 3.4.5.6-Tetrahydrophthalsäureanhydrid und einem entsprechenen Anilin der Formel III z.B. in einem Lösungsmittel bei einer Temperatur von 20 bis 200 ° C, vorzugsweise 40 bis 150 ° C, erhältlich. Als Lösungsmittel eignen sich z.B. niedere Alkansäuren wie Eisessig oder Propionsäure oder aprotische Lösungsmittel wie Toluol oder Xylol in Gegenwart eines sauren Katalysators wie z.B. einer aromatischen Sulfonsäure.

Unter den Verbindungen I sind diejenigen bevorzugt, in denen $R^1$ Wasserstoff oder Fluor, $R^2$ und $R^3$ Wasserstoff oder Methyl, $R^4$ Wasserstoff und $R^5$ 2- oder 3-Tetrahydrofuryl, 2- oder 3-Tetrahydrothienyl, 2-, 3- oder 4-Tetrahydropyranyl, 2-, 3- oder 4-Tetrahydrothiopyranyl, 5,6-Dihydro-2H-pyranyl und 5,6-Dihydro-2H-thiopyranyl bedeutet.

Besonders aktive Vertreter der erfindungsgemäßen Verbindungen sind in den nachstehenden Tabellen I, II, IIa, III, IIIa, IV, IVa, V und VI aufgeführt.

Tabelle I

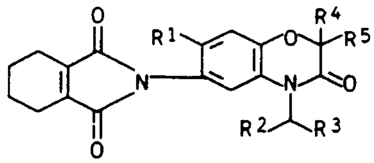

| $R^1$ | $R^2$ | $R^3$ | $R^4$ | $R^5$ |
|---|---|---|---|---|
| H | H | 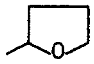 | H | H |
| F | H | 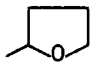 | H | H |
| H | $CH_3$ | 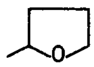 | H | H |
| F | $CH_3$ | 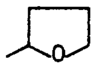 | H | H |
| H | H | 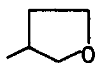 | H | H |
| F | H | 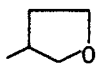 | H | H |
| H | $CH_3$ | 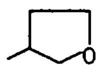 | H | H |
| F | $CH_3$ | 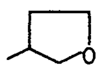 | H | H |
| H | H | 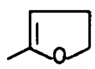 | H | H |
| F | H | 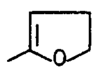 | H | H |
| H | $CH_3$ | 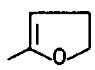 | H | H |
| F | $CH_3$ | 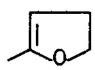 | H | H |
| H | H | 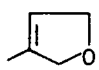 | H | H |
| F | H | 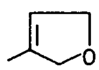 | H | H |

Tabelle I (Fortsetzung)

| $R^1$ | $R^2$ | $R^3$ | $R^4$ | $R^5$ |
|---|---|---|---|---|
| H | $CH_3$ | 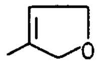 | H | H |
| F | $CH_3$ | 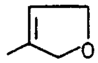 | H | H |
| H | H | 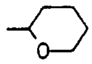 | H | H |
| F | H | 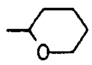 | H | H |
| H | $CH_3$ | 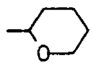 | H | H |
| F | $CH_3$ | 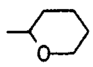 | H | H |
| H | H | 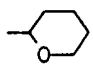 | $CH_3$ | H |
| F | H | 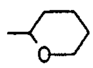 | $CH_3$ | H |
| H | $CH_3$ | 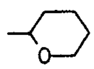 | $CH_3$ | H |
| F | $CH_3$ | 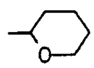 | $CH_3$ | H |
| H | H | 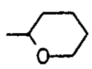 | $CH_3$ | $CH_3$ |
| F | H | 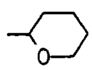 | $CH_3$ | $CH_3$ |
| H | $CH_3$ | 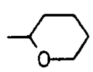 | $CH_3$ | $CH_3$ |
| F | $CH_3$ | 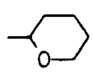 | $CH_3$ | $CH_3$ |
| H | H | 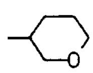 | H | H |
| F | H | 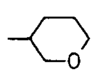 | H | H |
| H | $CH_3$ | 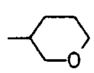 | H | H |

Tabelle I (Fortsetzung)

| $R^1$ | $R^2$ | $R^3$ | $R^4$ | $R^5$ |
|---|---|---|---|---|
| F | $CH_3$ | 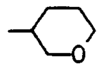 | H | H |
| H | H | 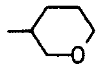 | $CH_3$ | H |
| F | H | 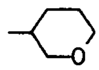 | $CH_3$ | H |
| H | $CH_3$ | 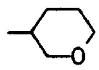 | $CH_3$ | H |
| F | $CH_3$ | 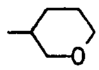 | $CH_3$ | H |
| H | H | 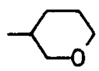 | $CH_3$ | $CH_3$ |
| F | H | 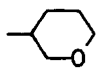 | $CH_3$ | $CH_3$ |
| H | $CH_3$ | 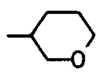 | $CH_3$ | $CH_3$ |
| F | $CH_3$ | 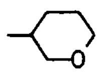 | $CH_3$ | $CH_3$ |
| H | H | 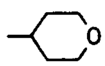 | H | H |
| F | H | 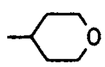 | H | H |
| H | $CH_3$ | 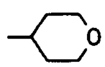 | H | H |
| F | $CH_3$ | 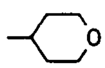 | H | H |
| H | H | 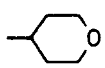 | $CH_3$ | H |
| F | H | 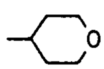 | $CH_3$ | H |
| H | $CH_3$ | 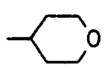 | $CH_3$ | H |

Tabelle I (Fortsetzung)

| $R^1$ | $R^2$ | $R^3$ | $R^4$ | $R^5$ |
|---|---|---|---|---|
| F | $CH_3$ | 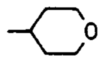 | $CH_3$ | H |
| H | H | 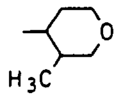 | H | H |
| F | H | 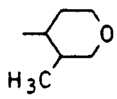 | H | H |
| H | $CH_3$ | 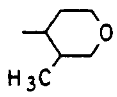 | H | H |
| F | $CH_3$ | 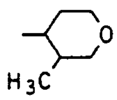 | H | H |
| H | H | 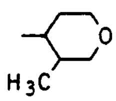 | $CH_3$ | H |
| F | H | 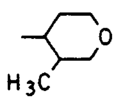 | $CH_3$ | H |
| H | $CH_3$ | 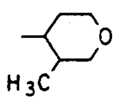 | $CH_3$ | H |
| F | $CH_3$ | 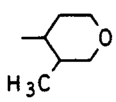 | $CH_3$ | H |
| H | H | 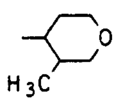 | $CH_3$ | $CH_3$ |
| F | H | 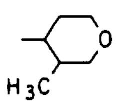 | $CH_3$ | $CH_3$ |
| H | $CH_3$ | 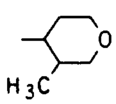 | $CH_3$ | $CH_3$ |
| F | $CH_3$ | 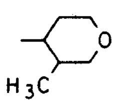 | $CH_3$ | $CH_3$ |

Tabelle I (Fortsetzung)

| $R^1$ | $R^2$ | $R^3$ | $R^4$ | $R^5$ |
|---|---|---|---|---|
| H | H | 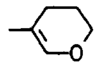 | H | H |
| F | H | 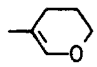 | H | H |
| H | $CH_3$ | 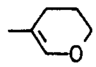 | H | H |
| F | $CH_3$ | 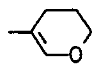 | H | H |
| H | H | 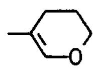 | $CH_3$ | H |
| F | H | 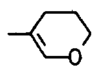 | $CH_3$ | H |
| H | $CH_3$ | 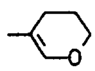 | $CH_3$ | H |
| F | $CH_3$ | 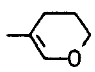 | $CH_3$ | H |
| H | H | 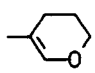 | $CH_3$ | $CH_3$ |
| F | H | 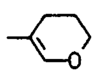 | $CH_3$ | $CH_3$ |
| H | $CH_3$ | 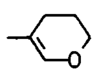 | $CH_3$ | $CH_3$ |
| F | $CH_3$ | 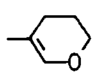 | $CH_3$ | $CH_3$ |
| H | H | 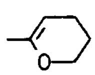 | $CH_3$ | $CH_3$ |
| F | H | 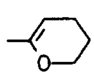 | $CH_3$ | $CH_3$ |
| H | $CH_3$ | 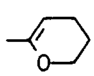 | $CH_3$ | $CH_3$ |
| F | $CH_3$ | 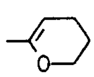 | $CH_3$ | $CH_3$ |

Tabelle I (Fortsetzung)

| $R^1$ | $R^2$ | $R^3$ | $R^4$ | $R^5$ |
|---|---|---|---|---|
| H | H | 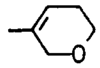 | H | H |
| F | H | 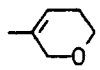 | H | H |
| H | $CH_3$ | 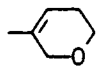 | H | H |
| F | $CH_3$ | 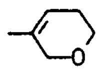 | H | H |
| H | H | 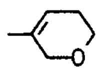 | $CH_3$ | H |
| F | H | 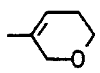 | $CH_3$ | H |
| H | $CH_3$ | 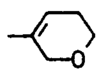 | $CH_3$ | H |
| F | $CH_3$ | 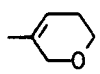 | $CH_3$ | H |
| H | H | 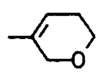 | $CH_3$ | $CH_3$ |
| F | H | 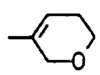 | $CH_3$ | $CH_3$ |
| H | $CH_3$ | 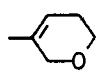 | $CH_3$ | $CH_3$ |
| F | $CH_3$ | 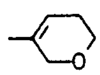 | $CH_3$ | $CH_3$ |
| H | H | 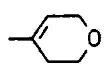 | H | H |
| F | H | 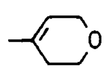 | H | H |
| H | $CH_3$ | 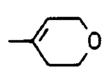 | H | H |
| F | $CH_3$ | 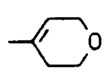 | H | H |

Tabelle I (Fortsetzung)

| $R^1$ | $R^2$ | $R^3$ | $R^4$ | $R^5$ |
|---|---|---|---|---|
| H | H | 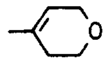 | $CH_3$ | H |
| F | H | 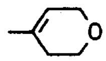 | $CH_3$ | H |
| H | $CH_3$ | 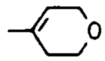 | $CH_3$ | H |
| F | $CH_3$ | 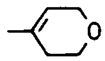 | $CH_3$ | H |
| H | H | 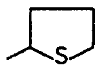 | H | H |
| F | H | 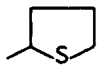 | H | H |
| H | $CH_3$ | 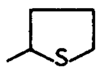 | H | H |
| F | $CH_3$ | 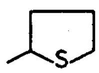 | H | H |
| H | H | 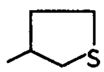 | H | H |
| F | H | 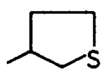 | H | H |
| H | $CH_3$ | 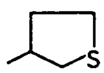 | H | H |
| F | $CH_3$ | 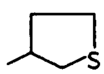 | H | H |
| H | H | 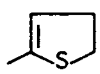 | H | H |
| F | H | 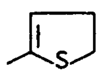 | H | H |
| H | $CH_3$ | 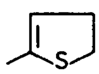 | H | H |
| F | $CH_3$ | 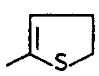 | H | H |

Tabelle I (Fortsetzung)

| $R^1$ | $R^2$ | $R^3$ | $R^4$ | $R^5$ |
|---|---|---|---|---|
| H | H | 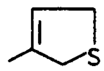 | H | H |
| F | H | 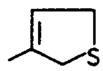 | H | H |
| H | $CH_3$ | 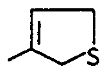 | H | H |
| F | $CH_3$ | 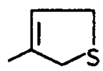 | H | H |
| H | H | 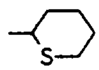 | H | H |
| F | H | 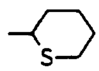 | H | H |
| H | $CH_3$ | 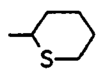 | H | H |
| F | $CH_3$ | 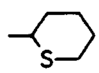 | H | H |
| H | H | 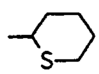 | $CH_3$ | H |
| F | H | 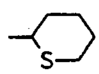 | $CH_3$ | H |
| H | $CH_3$ | 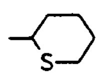 | $CH_3$ | H |
| F | $CH_3$ | 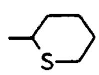 | $CH_3$ | H |
| H | H | 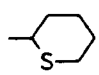 | $CH_3$ | $CH_3$ |
| F | H | 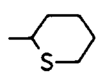 | $CH_3$ | $CH_3$ |
| H | $CH_3$ | 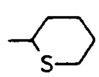 | $CH_3$ | $CH_3$ |
| F | $CH_3$ | 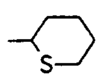 | $CH_3$ | $CH_3$ |

Tabelle I (Fortsetzung)

| $R^1$ | $R^2$ | $R^3$ | $R^4$ | $R^5$ |
|---|---|---|---|---|
| H | H | 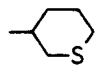 | H | H |
| F | H | 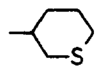 | H | H |
| H | $CH_3$ | 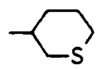 | H | H |
| F | $CH_3$ | 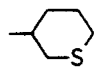 | H | H |
| H | H | 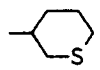 | $CH_3$ | H |
| F | H | 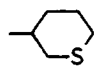 | $CH_3$ | H |
| H | $CH_3$ | 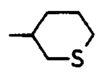 | $CH_3$ | H |
| F | $CH_3$ | 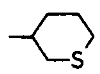 | $CH_3$ | H |
| H | H | 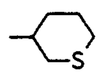 | $CH_3$ | $CH_3$ |
| F | H | 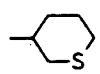 | $CH_3$ | $CH_3$ |
| H | $CH_3$ | 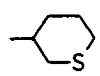 | $CH_3$ | $CH_3$ |
| F | $CH_3$ | 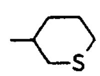 | $CH_3$ | $CH_3$ |
| H | H | 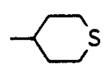 | H | H |
| F | H | 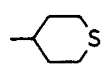 | H | H |
| H | $CH_3$ | 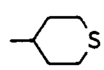 | H | H |
| F | $CH_3$ | 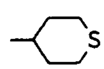 | H | H |

Tabelle I (Fortsetzung)

| $R^1$ | $R^2$ | $R^3$ | $R^4$ | $R^5$ |
|---|---|---|---|---|
| H | H | 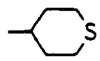 | $CH_3$ | H |
| F | H | 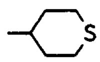 | $CH_3$ | H |
| H | $CH_3$ | 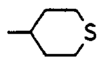 | $CH_3$ | H |
| F | $CH_3$ | 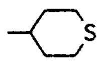 | $CH_3$ | H |
| H | H | 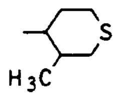 | H | H |
| F | H | 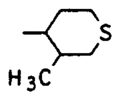 | H | H |
| H | $CH_3$ | 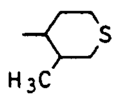 | H | H |
| F | $CH_3$ | 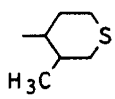 | H | H |
| H | H | 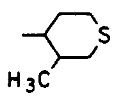 | $CH_3$ | H |
| F | H | 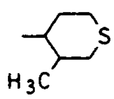 | $CH_3$ | H |
| H | $CH_3$ | 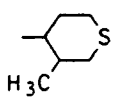 | $CH_3$ | H |
| F | $CH_3$ | 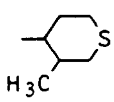 | $CH_3$ | H |
| H | H | 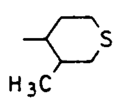 | $CH_3$ | $CH_3$ |
| F | H | 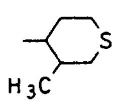 | $CH_3$ | $CH_3$ |

Tabelle I (Fortsetzung)

| $R^1$ | $R^2$ | $R^3$ | $R^4$ | $R^5$ |
|---|---|---|---|---|
| H | $CH_3$ | 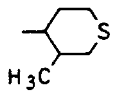 | $CH_3$ | $CH_3$ |
| F | $CH_3$ | 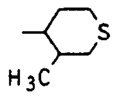 | $CH_3$ | $CH_3$ |
| H | H | 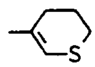 | H | H |
| F | H | 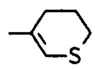 | H | H |
| H | $CH_3$ | 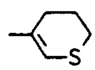 | H | H |
| F | $CH_3$ | 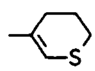 | H | H |
| H | H | 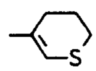 | $CH_3$ | H |
| F | H | 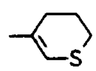 | $CH_3$ | H |
| H | $CH_3$ | 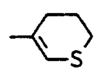 | $CH_3$ | H |
| F | $CH_3$ | 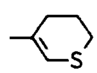 | $CH_3$ | H |
| H | H | 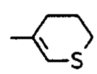 | $CH_3$ | $CH_3$ |
| F | H | 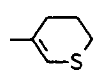 | $CH_3$ | $CH_3$ |
| H | $CH_3$ | 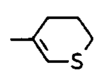 | $CH_3$ | $CH_3$ |
| F | $CH_3$ | 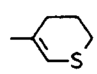 | $CH_3$ | $CH_3$ |
| H | H | 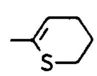 | $CH_3$ | $CH_3$ |
| F | H | 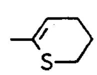 | $CH_3$ | $CH_3$ |

Tabelle I (Fortsetzung)

| $R^1$ | $R^2$ | $R^3$ | $R^4$ | $R^5$ |
|---|---|---|---|---|
| H | $CH_3$ | 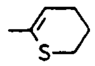 | $CH_3$ | $CH_3$ |
| F | $CH_3$ | 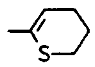 | $CH_3$ | $CH_3$ |
| H | H | 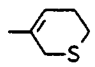 | H | H |
| F | H | 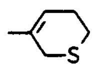 | H | H |
| H | $CH_3$ | 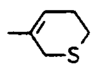 | H | H |
| F | $CH_3$ | 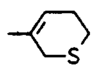 | H | H |
| H | H | 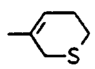 | $CH_3$ | H |
| F | H | 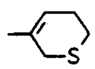 | $CH_3$ | H |
| H | $CH_3$ | 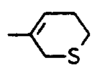 | $CH_3$ | H |
| F | $CH_3$ | 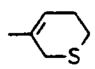 | $CH_3$ | H |
| H | H | 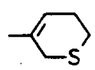 | $CH_3$ | $CH_3$ |
| F | H | 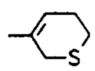 | $CH_3$ | $CH_3$ |
| H | $CH_3$ | 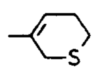 | $CH_3$ | $CH_3$ |
| F | $CH_3$ | 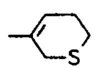 | $CH_3$ | $CH_3$ |
| H | H | 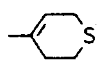 | H | H |
| F | H | 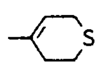 | H | H |
| H | $CH_3$ | 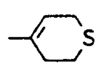 | H | H |

Tabelle I (Fortsetzung)

| $R^1$ | $R^2$ | $R^3$ | $R^4$ | $R^5$ |
|---|---|---|---|---|
| F | $CH_3$ | 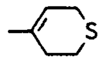 | H | H |
| H | H | 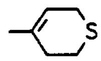 | $CH_3$ | H |
| F | H | 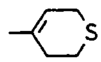 | $CH_3$ | H |
| H | $CH_3$ | 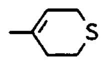 | $CH_3$ | H |
| F | $CH_3$ | 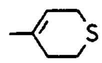 | $CH_3$ | H |
| H | H | 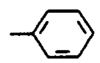 | H | H |
| F | H | 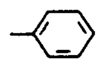 | H | H |
| H | $CH_3$ | 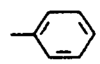 | H | H |
| F | $CH_3$ | 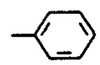 | H | H |
| H | H | 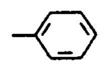 | $CH_3$ | H |
| F | H | 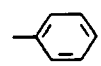 | $CH_3$ | H |
| H | $CH_3$ | 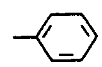 | $CH_3$ | H |
| F | $CH_3$ | 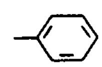 | $CH_3$ | H |
| H | H | 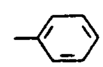 | $CH_3$ | $CH_3$ |
| F | H | 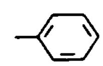 | $CH_3$ | $CH_3$ |
| H | $CH_3$ | 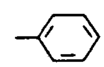 | $CH_3$ | $CH_3$ |

Tabelle I (Fortsetzung)

| $R^1$ | $R^2$ | $R^3$ | $R^4$ | $R^5$ |
|---|---|---|---|---|
| F | $CH_3$ | 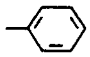 | $CH_3$ | $CH_3$ |
| H | H | 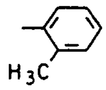 | H | H |
| F | H | 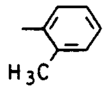 | H | H |
| H | $CH_3$ | 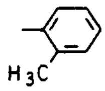 | H | H |
| F | $CH_3$ | 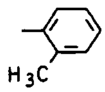 | H | H |
| H | H | 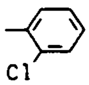 | H | H |
| F | H | 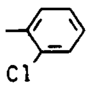 | H | H |
| H | $CH_3$ | 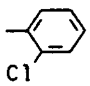 | H | H |
| F | $CH_3$ | 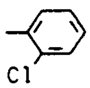 | H | H |
| H | H | 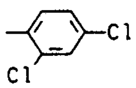 | H | H |
| F | H | 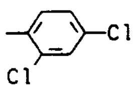 | H | H |
| H | $CH_3$ | 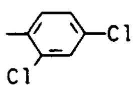 | H | H |
| F | $CH_3$ | 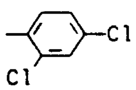 | H | H |

Tabelle II

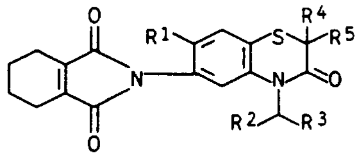

| $R^1$ | $R^2$ | $R^3$ | $R^4$ | $R^5$ |
|---|---|---|---|---|
| H | H | 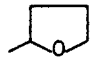 | H | H |
| F | H | 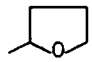 | H | H |
| H | $CH_3$ | 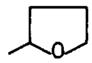 | H | H |
| F | $CH_3$ | 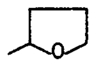 | H | H |
| H | H | 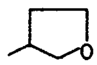 | H | H |
| F | H | 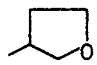 | H | H |
| H | $CH_3$ | 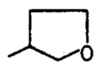 | H | H |
| F | $CH_3$ | 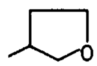 | H | H |
| H | H | 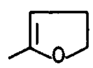 | H | H |
| F | H | 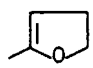 | H | H |
| H | $CH_3$ | 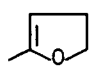 | H | H |
| F | $CH_3$ | 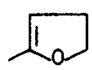 | H | H |
| H | H | 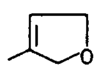 | H | H |
| F | H | 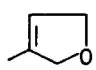 | H | H |

Tabelle II (Fortsetzung)

| $R^1$ | $R^2$ | $R^3$ | $R^4$ | $R^5$ |
|---|---|---|---|---|
| H | $CH_3$ | 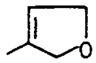 | H | H |
| F | $CH_3$ | 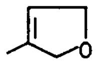 | H | H |
| H | H | 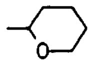 | H | H |
| F | H | 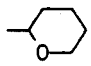 | H | H |
| H | $CH_3$ | 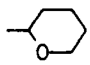 | H | H |
| F | $CH_3$ | 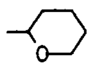 | H | H |
| H | H | 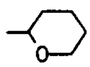 | $CH_3$ | H |
| F | H | 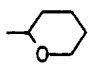 | $CH_3$ | H |
| H | $CH_3$ | 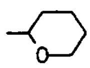 | $CH_3$ | H |
| F | $CH_3$ | 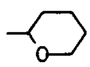 | $CH_3$ | H |
| H | H | 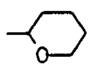 | $CH_3$ | $CH_3$ |
| F | H | 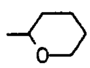 | $CH_3$ | $CH_3$ |
| H | $CH_3$ | 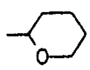 | $CH_3$ | $CH_3$ |
| F | $CH_3$ | 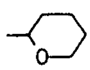 | $CH_3$ | $CH_3$ |
| H | H | 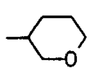 | H | H |
| F | H | 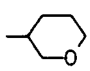 | H | H |
| H | $CH_3$ | 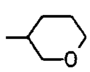 | H | H |

Tabelle II (Fortsetzung)

| $R^1$ | $R^2$ | $R^3$ | $R^4$ | $R^5$ |
|---|---|---|---|---|
| F | $CH_3$ | 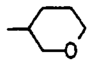 | H | H |
| H | H | 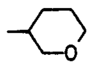 | $CH_3$ | H |
| F | H | 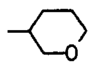 | $CH_3$ | H |
| H | $CH_3$ | 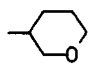 | $CH_3$ | H |
| F | $CH_3$ | 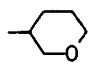 | $CH_3$ | H |
| H | H | 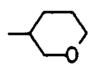 | $CH_3$ | $CH_3$ |
| F | H | 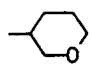 | $CH_3$ | $CH_3$ |
| H | $CH_3$ | 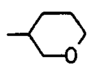 | $CH_3$ | $CH_3$ |
| F | $CH_3$ | 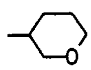 | $CH_3$ | $CH_3$ |
| H | H | 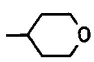 | H | H |
| F | H | 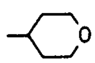 | H | H |
| H | $CH_3$ | 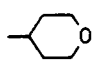 | H | H |
| F | $CH_3$ | 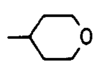 | H | H |
| H | H | 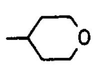 | $CH_3$ | H |
| F | H | 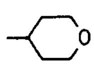 | $CH_3$ | H |
| H | $CH_3$ | 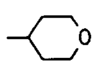 | $CH_3$ | H |

Tabelle II (Fortsetzung)

| $R^1$ | $R^2$ | $R^3$ | $R^4$ | $R^5$ |
|---|---|---|---|---|
| F | $CH_3$ | 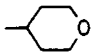 | $CH_3$ | H |
| H | H | 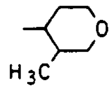 | H | H |
| F | H | 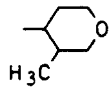 | H | H |
| H | $CH_3$ | 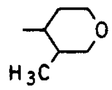 | H | H |
| F | $CH_3$ | 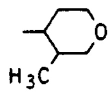 | H | H |
| H | H | 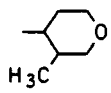 | $CH_3$ | H |
| F | H | 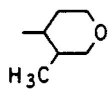 | $CH_3$ | H |
| H | $CH_3$ | 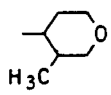 | $CH_3$ | H |
| F | $CH_3$ | 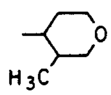 | $CH_3$ | H |
| H | H | 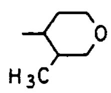 | $CH_3$ | $CH_3$ |
| F | H | 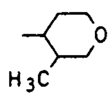 | $CH_3$ | $CH_3$ |
| H | $CH_3$ | 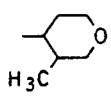 | $CH_3$ | $CH_3$ |
| F | $CH_3$ | 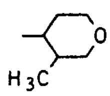 | $CH_3$ | $CH_3$ |

Tabelle II (Fortsetzung)

| $R^1$ | $R^2$ | $R^3$ | $R^4$ | $R^5$ |
|---|---|---|---|---|
| H | H | 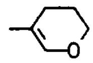 | H | H |
| F | H | 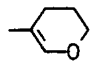 | H | H |
| H | $CH_3$ | 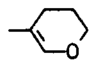 | H | H |
| F | $CH_3$ | 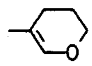 | H | H |
| H | H | 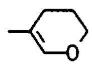 | $CH_3$ | H |
| F | H | 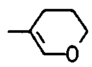 | $CH_3$ | H |
| H | $CH_3$ | 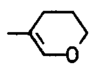 | $CH_3$ | H |
| F | $CH_3$ | 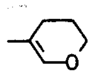 | $CH_3$ | H |
| H | H | 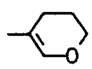 | $CH_3$ | $CH_3$ |
| F | H | 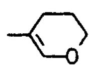 | $CH_3$ | $CH_3$ |
| H | $CH_3$ | 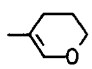 | $CH_3$ | $CH_3$ |
| F | $CH_3$ | 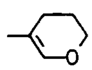 | $CH_3$ | $CH_3$ |
| H | H | 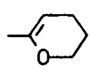 | $CH_3$ | $CH_3$ |
| F | H | 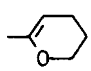 | $CH_3$ | $CH_3$ |
| H | $CH_3$ | 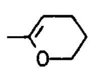 | $CH_3$ | $CH_3$ |
| F | $CH_3$ | 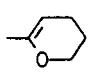 | $CH_3$ | $CH_3$ |

Tabelle II (Fortsetzung)

| $R^1$ | $R^2$ | $R^3$ | $R^4$ | $R^5$ |
|---|---|---|---|---|
| H | H | 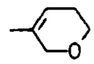 | H | H |
| F | H | 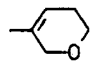 | H | H |
| H | $CH_3$ | 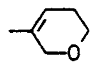 | H | H |
| F | $CH_3$ | 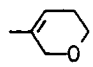 | H | H |
| H | H | 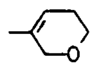 | $CH_3$ | H |
| F | H | 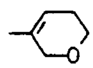 | $CH_3$ | H |
| H | $CH_3$ | 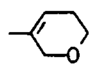 | $CH_3$ | H |
| F | $CH_3$ | 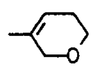 | $CH_3$ | H |
| H | H | 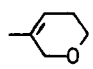 | $CH_3$ | $CH_3$ |
| F | H | 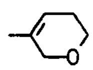 | $CH_3$ | $CH_3$ |
| H | $CH_3$ | 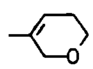 | $CH_3$ | $CH_3$ |
| F | $CH_3$ | 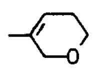 | $CH_3$ | $CH_3$ |
| H | H | 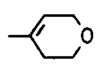 | H | H |
| F | H | 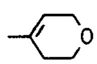 | H | H |
| H | $CH_3$ | 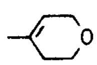 | H | H |
| F | $CH_3$ | 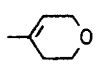 | H | H |

Tabelle II (Fortsetzung)

| $R^1$ | $R^2$ | $R^3$ | $R^4$ | $R^5$ |
|---|---|---|---|---|
| H | H | 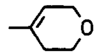 | $CH_3$ | H |
| F | H | 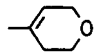 | $CH_3$ | H |
| H | $CH_3$ | 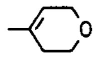 | $CH_3$ | H |
| F | $CH_3$ | 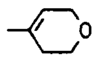 | $CH_3$ | H |
| H | H | 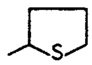 | H | H |
| F | H | 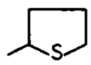 | H | H |
| H | $CH_3$ | 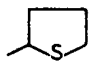 | H | H |
| F | $CH_3$ | 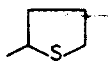 | H | H |
| H | H | 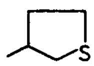 | H | H |
| F | H | 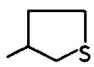 | H | H |
| H | $CH_3$ | 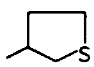 | H | H |
| F | $CH_3$ | 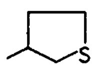 | H | H |
| H | H | 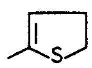 | H | H |
| F | H | 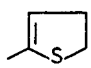 | H | H |
| H | $CH_3$ | 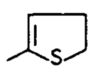 | H | H |
| F | $CH_3$ | 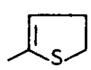 | H | H |

Tabelle II (Fortsetzung)

| $R^1$ | $R^2$ | $R^3$ | $R^4$ | $R^5$ |
|---|---|---|---|---|
| H | H | 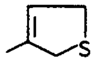 | H | H |
| F | H | 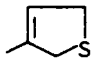 | H | H |
| H | $CH_3$ | 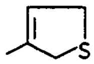 | H | H |
| F | $CH_3$ | 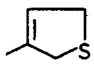 | H | H |
| H | H | 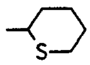 | H | H |
| F | H | 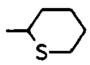 | H | H |
| H | $CH_3$ | 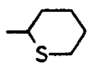 | H | H |
| F | $CH_3$ | 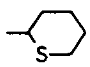 | H | H |
| H | H | 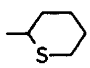 | $CH_3$ | H |
| F | H | 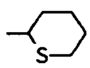 | $CH_3$ | H |
| H | $CH_3$ | 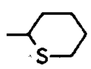 | $CH_3$ | H |
| F | $CH_3$ | 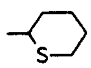 | $CH_3$ | H |
| H | H | 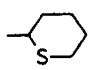 | $CH_3$ | $CH_3$ |
| F | H | 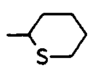 | $CH_3$ | $CH_3$ |
| H | $CH_3$ | 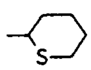 | $CH_3$ | $CH_3$ |
| F | $CH_3$ | 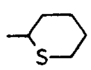 | $CH_3$ | $CH_3$ |

Tabelle II (Fortsetzung)

| $R^1$ | $R^2$ | $R^3$ | $R^4$ | $R^5$ |
|---|---|---|---|---|
| H | H | 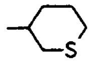 | H | H |
| F | H | 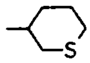 | H | H |
| H | $CH_3$ | 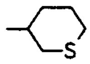 | H | H |
| F | $CH_3$ | 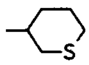 | H | H |
| H | H | 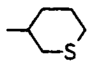 | $CH_3$ | H |
| F | H | 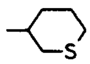 | $CH_3$ | H |
| H | $CH_3$ | 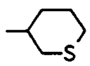 | $CH_3$ | H |
| F | $CH_3$ | 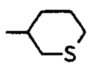 | $CH_3$ | H |
| H | H | 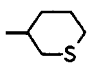 | $CH_3$ | $CH_3$ |
| F | H | 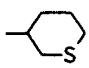 | $CH_3$ | $CH_3$ |
| H | $CH_3$ | 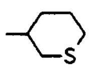 | $CH_3$ | $CH_3$ |
| F | $CH_3$ | 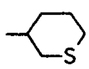 | $CH_3$ | $CH_3$ |
| H | H | 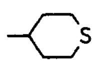 | H | H |
| F | H | 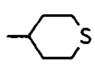 | H | H |
| H | $CH_3$ | 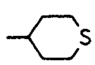 | H | H |
| F | $CH_3$ | 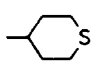 | H | H |

Tabelle II (Fortsetzung)

| $R^1$ | $R^2$ | $R^3$ | $R^4$ | $R^5$ |
|---|---|---|---|---|
| H | H | 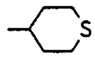 | $CH_3$ | H |
| F | H | 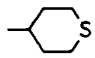 | $CH_3$ | H |
| H | $CH_3$ | 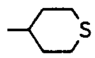 | $CH_3$ | H |
| F | $CH_3$ | 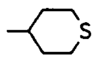 | $CH_3$ | H |
| H | H | 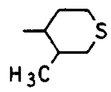 | H | H |
| F | H | 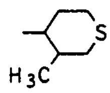 | H | H |
| H | $CH_3$ | 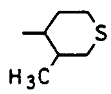 | H | H |
| F | $CH_3$ | 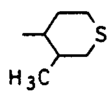 | H | H |
| H | H | 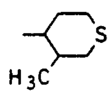 | $CH_3$ | H |
| F | H | 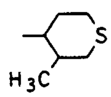 | $CH_3$ | H |
| H | $CH_3$ | 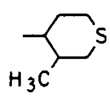 | $CH_3$ | H |
| F | $CH_3$ | 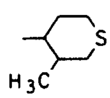 | $CH_3$ | H |
| H | H | 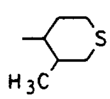 | $CH_3$ | $CH_3$ |
| F | H | 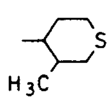 | $CH_3$ | $CH_3$ |

Tabelle II (Fortsetzung)

| $R^1$ | $R^2$ | $R^3$ | $R^4$ | $R^5$ |
|---|---|---|---|---|
| H | $CH_3$ | 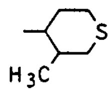 | $CH_3$ | $CH_3$ |
| F | $CH_3$ | 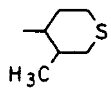 | $CH_3$ | $CH_3$ |
| H | H | 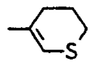 | H | H |
| F | H | 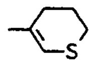 | H | H |
| H | $CH_3$ | 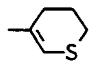 | H | H |
| F | $CH_3$ | 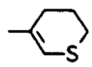 | H | H |
| H | H | 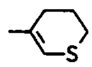 | $CH_3$ | H |
| F | H | 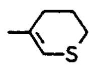 | $CH_3$ | H |
| H | $CH_3$ | 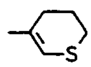 | $CH_3$ | H |
| F | $CH_3$ | 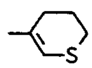 | $CH_3$ | H |
| H | H | 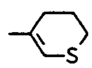 | $CH_3$ | $CH_3$ |
| F | H | 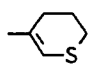 | $CH_3$ | $CH_3$ |
| H | $CH_3$ | 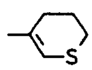 | $CH_3$ | $CH_3$ |
| F | $CH_3$ | 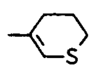 | $CH_3$ | $CH_3$ |
| H | H | 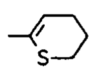 | $CH_3$ | $CH_3$ |
| F | H | 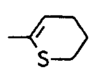 | $CH_3$ | $CH_3$ |

Tabelle II (Fortsetzung)

| $R^1$ | $R^2$ | $R^3$ | $R^4$ | $R^5$ |
|---|---|---|---|---|
| H | $CH_3$ | 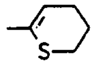 | $CH_3$ | $CH_3$ |
| F | $CH_3$ | 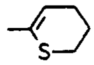 | $CH_3$ | $CH_3$ |
| H | H | 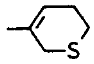 | H | H |
| F | H | 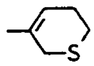 | H | H |
| H | $CH_3$ | 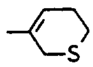 | H | H |
| F | $CH_3$ | 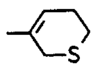 | H | H |
| H | H | 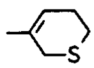 | $CH_3$ | H |
| F | H | 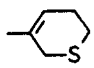 | $CH_3$ | H |
| H | $CH_3$ | 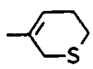 | $CH_3$ | H |
| F | $CH_3$ | 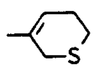 | $CH_3$ | H |
| H | H | 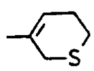 | $CH_3$ | $CH_3$ |
| F | H | 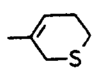 | $CH_3$ | $CH_3$ |
| H | $CH_3$ | 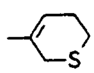 | $CH_3$ | $CH_3$ |
| F | $CH_3$ | 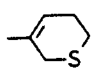 | $CH_3$ | $CH_3$ |
| H | H | 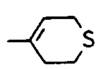 | H | H |
| F | H | 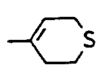 | H | H |

Tabelle II (Fortsetzung)

| $R^1$ | $R^2$ | $R^3$ | $R^4$ | $R^5$ |
|---|---|---|---|---|
| H | $CH_3$ | 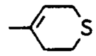 | H | H |
| F | $CH_3$ | 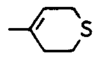 | H | H |
| H | H | 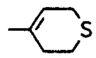 | $CH_3$ | H |
| F | H | 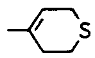 | $CH_3$ | H |
| H | $CH_3$ | 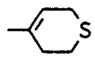 | $CH_3$ | H |
| F | $CH_3$ | 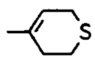 | $CH_3$ | H |
| H | H | 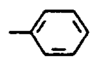 | H | H |
| F | H | 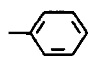 | H | H |
| H | $CH_3$ | 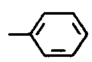 | H | H |
| F | $CH_3$ | 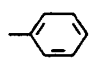 | H | H |
| H | H | 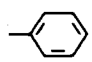 | $CH_3$ | H |
| F | H | 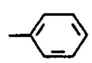 | $CH_3$ | H |
| H | $CH_3$ | 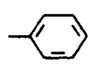 | $CH_3$ | H |
| F | $CH_3$ | 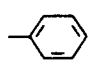 | $CH_3$ | H |
| H | H | 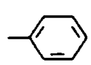 | $CH_3$ | $CH_3$ |
| F | H | 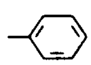 | $CH_3$ | $CH_3$ |

Tabelle II (Fortsetzung)

| $R^1$ | $R^2$ | $R^3$ | $R^4$ | $R^5$ |
|---|---|---|---|---|
| H | $CH_3$ | 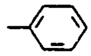 | $CH_3$ | $CH_3$ |
| F | $CH_3$ | 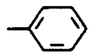 | $CH_3$ | $CH_3$ |
| H | H | 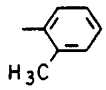 | H | H |
| F | H | 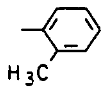 | H | H |
| H | $CH_3$ | 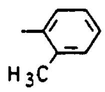 | H | H |
| F | $CH_3$ | 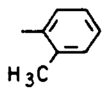 | H | H |
| H | H | 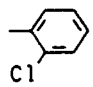 | H | H |
| F | H | 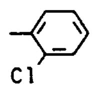 | H | H |
| H | $CH_3$ | 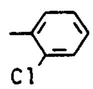 | H | H |
| F | $CH_3$ | 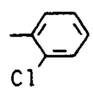 | H | H |
| H | H | 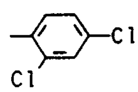 | H | H |
| F | H | 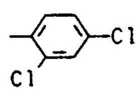 | H | H |
| H | $CH_3$ | 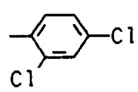 | H | H |
| F | $CH_3$ | 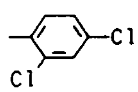 | H | H |

Tabelle IIa

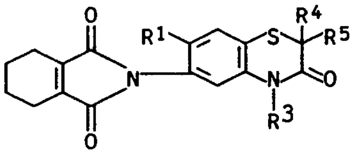

I (Y = $-S-CR^4R^5-$, n = 0)

| $R^1$ | $R^4$ | $R^5$ | $R^3$ |
|---|---|---|---|
| H | H | H | H |
| F | H | H | H |
| H | $CH_3$ | H | H |
| F | $CH_3$ | H | H |
| H | H | H | $CH_3$ |
| F | H | H | $CH_3$ |
| H | $CH_3$ | H | $CH_3$ |
| F | $CH_3$ | H | $CH_3$ |
| H | H | H | $CH_2CH_3$ |
| F | H | H | $CH_2CH_3$ |
| H | $CH_3$ | H | $CH_2CH_3$ |
| F | $CH_3$ | H | $CH_2CH_3$ |
| H | H | H | $CH(CH_3)_2$ |
| F | H | H | $CH(CH_3)_2$ |
| H | $CH_3$ | H | $CH(CH_3)_2$ |
| F | $CH_3$ | H | $CH(CH_3)_2$ |
| H | H | H | $CH_2CH=CH_2$ |
| F | H | H | $CH_2CH=CH_2$ |
| H | $CH_3$ | H | $CH_2CH=CH_2$ |
| F | $CH_3$ | H | $CH_2CH=CH_2$ |
| H | H | H | $CH_2CH=CHCH_3$ |
| F | H | H | $CH_2CH=CHCH_3$ |
| H | $CH_3$ | H | $CH_2CH=CHCH_3$ |
| F | $CH_3$ | H | $CH_2CH=CHCH_3$ |
| H | H | H | $CH_2C\equiv CH$ |
| F | H | H | $CH_2C\equiv CH$ |
| H | $CH_3$ | H | $CH_2C\equiv CH$ |
| F | $CH_3$ | H | $CH_2C\equiv CH$ |
| H | H | H | $CH_2C\equiv CCH_3$ |
| F | H | H | $CH_2C\equiv CCH_3$ |
| H | $CH_3$ | H | $CH_2C\equiv CCH_3$ |
| F | $CH_3$ | H | $CH_2C\equiv CCH_3$ |
| H | H | H | $CH_2OCH_3$ |
| F | H | H | $CH_2OCH_3$ |
| H | $CH_3$ | H | $CH_2OCH_3$ |
| F | $CH_3$ | H | $CH_2OCH_3$ |
| H | H | H | $(CH_2)_2OCH_3$ |
| F | H | H | $(CH_2)_2OCH_3$ |
| H | $CH_3$ | H | $(CH_2)_2OCH_3$ |
| F | $CH_3$ | H | $(CH_2)_2OCH_3$ |
| H | H | H | $CH_2CO_2CH_3$ |
| F | H | H | $CH_2CO_2CH_3$ |
| H | $CH_3$ | H | $CH_2CO_2CH_3$ |
| F | $CH_3$ | H | $CH_2CO_2CH_3$ |
| H | H | H | $CH_2CO_2CH_2CH_3$ |
| F | H | H | $CH_2CO_2CH_2CH_3$ |
| H | $CH_3$ | H | $CH_2CO_2CH_2CH_3$ |
| F | $CH_3$ | H | $CH_2CO_2CH_2CH_3$ |
| H | H | H | $CH(CH_3)CO_2CH_3$ |
| F | H | H | $CH(CH_3)CO_2CH_3$ |
| H | $CH_3$ | H | $CH(CH_3)CO_2CH_3$ |
| F | $CH_3$ | H | $CH(CH_3)CO_2CH_3$ |
| H | H | H | $CH(CH_3)CO_2CH_2CH_3$ |
| F | H | H | $CH(CH_3)CO_2CH_2CH_3$ |
| H | $CH_3$ | H | $CH(CH_3)CO_2CH_2CH_3$ |
| F | $CH_3$ | H | $CH(CH_3)CO_2CH_2CH_3$ |
| H | $CH_3$ | $CH_3$ | H |
| F | $CH_3$ | $CH_3$ | H |

Tabelle IIa (Fortsetzung)

| $R^1$ | $R^4$ | $R^5$ | $R^3$ |
|---|---|---|---|
| H | $CH_3$ | $CH_3$ | $CH_3$ |
| F | $CH_3$ | $CH_3$ | $CH_3$ |
| H | $CH_3$ | $CH_3$ | $CH_2CH_3$ |
| F | $CH_3$ | $CH_3$ | $CH_2CH_3$ |
| H | $CH_3$ | $CH_3$ | $CH_2CH{=}CH_2$ |
| F | $CH_3$ | $CH_3$ | $CH_2CH{=}CH_2$ |
| H | $CH_3$ | $CH_3$ | $CH_2C{\equiv}CH$ |
| F | $CH_3$ | $CH_3$ | $CH_2C{\equiv}CH$ |
| H | $CH_3$ | $CH_3$ | $(CH_2)_2OCH_3$ |
| F | $CH_3$ | $CH_3$ | $(CH_2)_2OCH_3$ |
| H | $CH_3$ | $CH_3$ | $CH_2CO_2CH_3$ |
| F | $CH_3$ | $CH_3$ | $CH_2CO_2CH_3$ |
| H | $CH_3$ | $CH_3$ | $CH(CH_3)CO_2CH_3$ |
| F | $CH_3$ | $CH_3$ | $CH(CH_3)CO_2CH_3$ |

Tabelle III

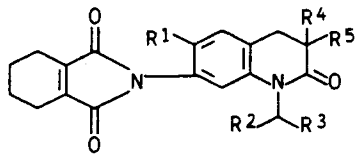

| R1 | R2 | R3 | R4 | R5 |
|---|---|---|---|---|
| H | H | 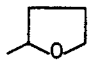 | H | H |
| F | H | 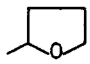 | H | H |
| H | $CH_3$ | 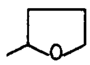 | H | H |
| F | $CH_3$ | 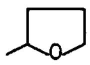 | H | H |
| H | H | 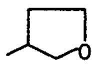 | H | H |
| F | H | 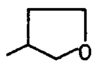 | H | H |
| H | $CH_3$ | 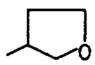 | H | H |
| F | $CH_3$ | 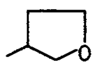 | H | H |
| H | H | 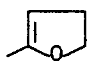 | H | H |
| F | H | 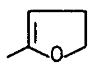 | H | H |
| H | $CH_3$ | 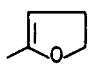 | H | H |
| F | $CH_3$ | 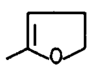 | H | H |
| H | H | 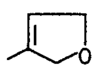 | H | H |
| F | H | 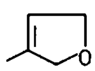 | H | H |

Tabelle III (Fortsetzung)

| $R^1$ | $R^2$ | $R^3$ | $R^4$ | $R^5$ |
|---|---|---|---|---|
| H | $CH_3$ | 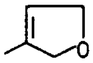 | H | H |
| F | $CH_3$ | 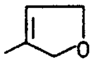 | H | H |
| H | H | 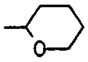 | H | H |
| F | H | 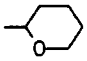 | H | H |
| H | $CH_3$ | 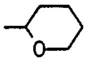 | H | H |
| F | $CH_3$ | 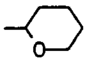 | H | H |
| H | H | 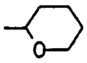 | $CH_3$ | H |
| F | H | 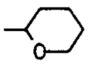 | $CH_3$ | H |
| H | $CH_3$ | 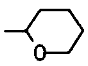 | $CH_3$ | H |
| F | $CH_3$ | 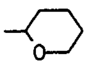 | $CH_3$ | H |
| H | H | 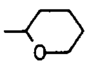 | $CH_3$ | $CH_3$ |
| F | H | 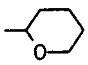 | $CH_3$ | $CH_3$ |
| H | $CH_3$ | 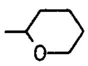 | $CH_3$ | $CH_3$ |
| F | $CH_3$ | 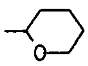 | $CH_3$ | $CH_3$ |
| H | H | 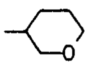 | H | H |
| F | H | 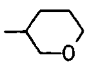 | H | H |

Tabelle III (Fortsetzung)

| $R^1$ | $R^2$ | $R^3$ | $R^4$ | $R^5$ |
|---|---|---|---|---|
| H | $CH_3$ | 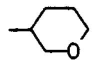 | H | H |
| F | $CH_3$ | 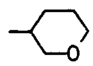 | H | H |
| H | H | 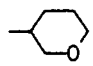 | $CH_3$ | H |
| F | H | 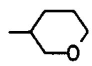 | $CH_3$ | H |
| H | $CH_3$ | 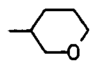 | $CH_3$ | H |
| F | $CH_3$ | 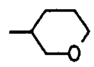 | $CH_3$ | H |
| H | H | 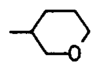 | $CH_3$ | $CH_3$ |
| F | H | 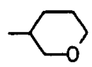 | $CH_3$ | $CH_3$ |
| H | $CH_3$ | 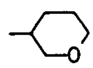 | $CH_3$ | $CH_3$ |
| F | $CH_3$ | 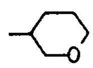 | $CH_3$ | $CH_3$ |
| H | H | 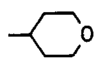 | H | H |
| F | H | 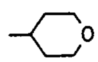 | H | H |
| H | $CH_3$ | 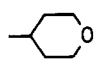 | H | H |
| F | $CH_3$ | 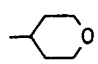 | H | H |
| H | H | 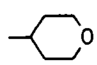 | $CH_3$ | H |
| F | H | 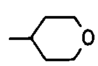 | $CH_3$ | H |

Tabelle III (Fortsetzung)

| $R^1$ | $R^2$ | $R^3$ | $R^4$ | $R^5$ |
|---|---|---|---|---|
| H | $CH_3$ | 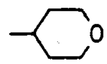 | $CH_3$ | H |
| F | $CH_3$ | 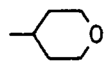 | $CH_3$ | H |
| H | H | 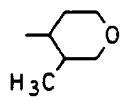 | H | H |
| F | H | 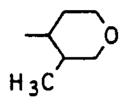 | H | H |
| H | $CH_3$ | 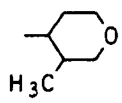 | H | H |
| F | $CH_3$ | 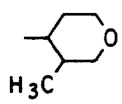 | H | H |
| H | H | 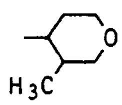 | $CH_3$ | H |
| F | H | 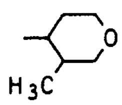 | $CH_3$ | H |
| H | $CH_3$ | 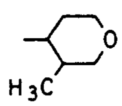 | $CH_3$ | H |
| F | $CH_3$ | 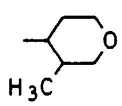 | $CH_3$ | H |
| H | H | 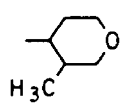 | $CH_3$ | $CH_3$ |
| F | H | 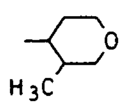 | $CH_3$ | $CH_3$ |
| H | $CH_3$ | 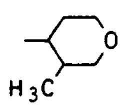 | $CH_3$ | $CH_3$ |

Tabelle III (Fortsetzung)

| $R^1$ | $R^2$ | $R^3$ | $R^4$ | $R^5$ |
|---|---|---|---|---|
| F | $CH_3$ | 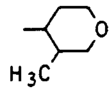 | $CH_3$ | $CH_3$ |
| H | H | 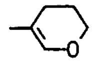 | H | H |
| F | H | 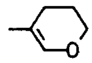 | H | H |
| H | $CH_3$ | 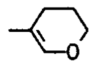 | H | H |
| F | $CH_3$ | 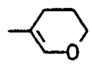 | H | H |
| H | H | 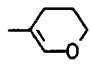 | $CH_3$ | H |
| F | H | 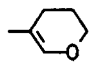 | $CH_3$ | H |
| H | $CH_3$ | 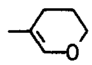 | $CH_3$ | H |
| F | $CH_3$ | 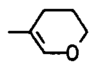 | $CH_3$ | H |
| H | H | 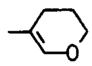 | $CH_3$ | $CH_3$ |
| F | H | 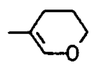 | $CH_3$ | $CH_3$ |
| H | $CH_3$ | 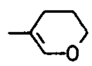 | $CH_3$ | $CH_3$ |
| F | $CH_3$ | 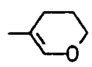 | $CH_3$ | $CH_3$ |
| H | H | 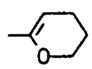 | $CH_3$ | $CH_3$ |
| F | H | 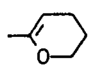 | $CH_3$ | $CH_3$ |
| H | $CH_3$ | 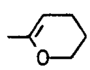 | $CH_3$ | $CH_3$ |

Tabelle III (Fortsetzung)

| $R^1$ | $R^2$ | $R^3$ | $R^4$ | $R^5$ |
|---|---|---|---|---|
| F | $CH_3$ | 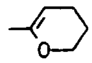 | $CH_3$ | $CH_3$ |
| H | H | 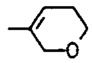 | H | H |
| F | H | 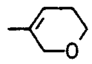 | H | H |
| H | $CH_3$ | 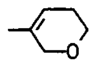 | H | H |
| F | $CH_3$ | 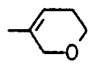 | H | H |
| H | H | 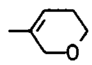 | $CH_3$ | H |
| F | H | 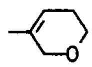 | $CH_3$ | H |
| H | $CH_3$ | 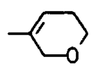 | $CH_3$ | H |
| F | $CH_3$ | 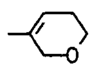 | $CH_3$ | H |
| H | H | 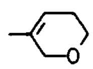 | $CH_3$ | $CH_3$ |
| F | H | 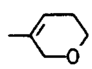 | $CH_3$ | $CH_3$ |
| H | $CH_3$ | 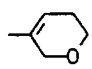 | $CH_3$ | $CH_3$ |
| F | $CH_3$ | 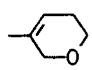 | $CH_3$ | $CH_3$ |
| H | H | 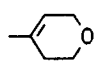 | H | H |
| F | H | 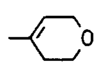 | H | H |
| H | $CH_3$ | 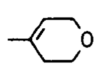 | H | H |

Tabelle III (Fortsetzung)

| $R^1$ | $R^2$ | $R^3$ | $R^4$ | $R^5$ |
|---|---|---|---|---|
| F | $CH_3$ | 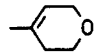 | H | H |
| H | H | 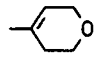 | $CH_3$ | H |
| F | H | 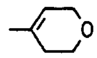 | $CH_3$ | H |
| H | $CH_3$ | 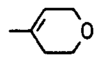 | $CH_3$ | H |
| F | $CH_3$ | 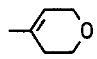 | $CH_3$ | H |
| H | H | 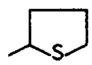 | H | H |
| F | H | 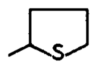 | H | H |
| H | $CH_3$ | 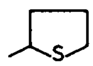 | H | H |
| F | $CH_3$ | 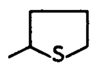 | H | H |
| H | H | 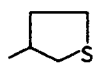 | H | H |
| F | H | 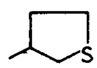 | H | H |
| H | $CH_3$ | 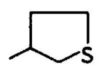 | H | H |
| F | $CH_3$ | 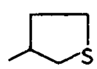 | H | H |
| H | H | 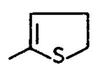 | H | H |
| F | H | 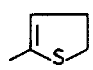 | H | H |
| H | $CH_3$ | 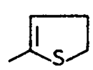 | H | H |

Tabelle III (Fortsetzung)

| $R^1$ | $R^2$ | $R^3$ | $R^4$ | $R^5$ |
|---|---|---|---|---|
| F | $CH_3$ | 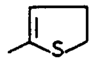 | H | H |
| H | H | 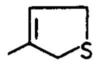 | H | H |
| F | H | 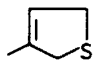 | H | H |
| H | $CH_3$ | 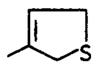 | H | H |
| F | $CH_3$ | 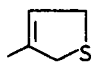 | H | H |
| H | H | 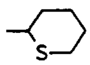 | H | H |
| F | H | 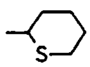 | H | H |
| H | $CH_3$ | 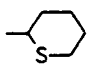 | H | H |
| F | $CH_3$ | 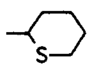 | H | H |
| H | H | 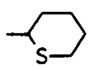 | $CH_3$ | H |
| F | H | 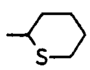 | $CH_3$ | H |
| H | $CH_3$ | 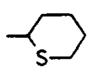 | $CH_3$ | H |
| F | $CH_3$ | 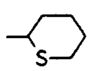 | $CH_3$ | H |
| H | H | 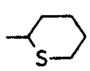 | $CH_3$ | $CH_3$ |
| F | H | 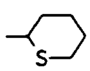 | $CH_3$ | $CH_3$ |
| H | $CH_3$ | 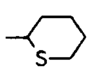 | $CH_3$ | $CH_3$ |

Tabelle III (Fortsetzung)

| $R^1$ | $R^2$ | $R^3$ | $R^4$ | $R^5$ |
|---|---|---|---|---|
| F | $CH_3$ | 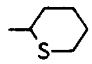 | $CH_3$ | $CH_3$ |
| H | H | 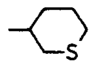 | H | H |
| F | H | 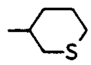 | H | H |
| H | $CH_3$ | 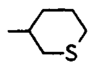 | H | H |
| F | $CH_3$ | 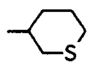 | H | H |
| H | H | 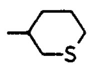 | $CH_3$ | H |
| F | H | 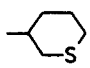 | $CH_3$ | H |
| H | $CH_3$ | 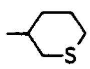 | $CH_3$ | H |
| F | $CH_3$ | 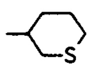 | $CH_3$ | H |
| H | H | 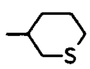 | $CH_3$ | $CH_3$ |
| F | H | 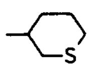 | $CH_3$ | $CH_3$ |
| H | $CH_3$ | 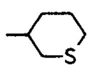 | $CH_3$ | $CH_3$ |
| F | $CH_3$ | 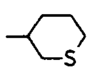 | $CH_3$ | $CH_3$ |
| H | H | 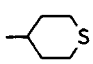 | H | H |
| F | H | 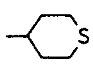 | H | H |
| H | $CH_3$ | 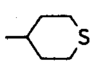 | H | H |

Tabelle III (Fortsetzung)

| $R^1$ | $R^2$ | $R^3$ | $R^4$ | $R^5$ |
|---|---|---|---|---|
| F | $CH_3$ | 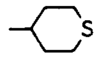 | H | H |
| H | H | 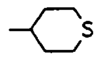 | $CH_3$ | H |
| F | H | 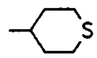 | $CH_3$ | H |
| H | $CH_3$ | 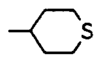 | $CH_3$ | H |
| F | $CH_3$ | 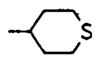 | $CH_3$ | H |
| H | H | 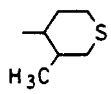 | H | H |
| F | H | 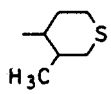 | H | H |
| F | $CH_3$ | 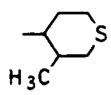 | H | H |
| F | $CH_3$ | 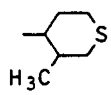 | H | H |
| H | H | 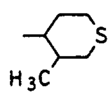 | $CH_3$ | H |
| F | H | 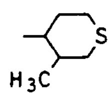 | $CH_3$ | H |
| H | $CH_3$ | 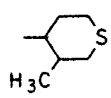 | $CH_3$ | H |
| F | $CH_3$ | 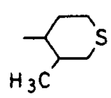 | $CH_3$ | H |
| H | H | 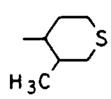 | $CH_3$ | $CH_3$ |

Tabelle III (Fortsetzung)

| $R^1$ | $R^2$ | $R^3$ | $R^4$ | $R^5$ |
|---|---|---|---|---|
| F | H | 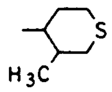 | $CH_3$ | $CH_3$ |
| H | $CH_3$ | 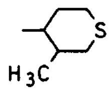 | $CH_3$ | $CH_3$ |
| F | $CH_3$ | 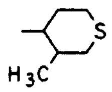 | $CH_3$ | $CH_3$ |
| H | H | 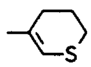 | H | H |
| F | H | 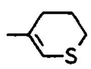 | H | H |
| H | $CH_3$ | 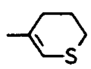 | H | H |
| F | $CH_3$ | 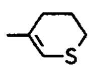 | H | H |
| H | H | 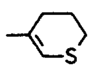 | $CH_3$ | H |
| F | H | 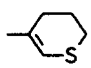 | $CH_3$ | H |
| H | $CH_3$ | 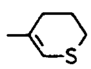 | $CH_3$ | H |
| F | $CH_3$ | 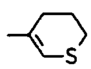 | $CH_3$ | H |
| H | H | 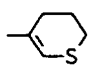 | $CH_3$ | $CH_3$ |
| F | H | 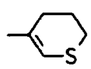 | $CH_3$ | $CH_3$ |
| H | $CH_3$ | 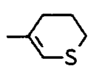 | $CH_3$ | $CH_3$ |
| F | $CH_3$ | 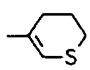 | $CH_3$ | $CH_3$ |
| H | H | 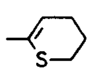 | $CH_3$ | $CH_3$ |

Tabelle III (Fortsetzung)

| $R^1$ | $R^2$ | $R^3$ | $R^4$ | $R^5$ |
|---|---|---|---|---|
| F | H | 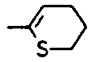 | $CH_3$ | $CH_3$ |
| H | $CH_3$ | 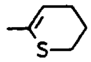 | $CH_3$ | $CH_3$ |
| F | $CH_3$ | 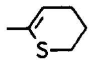 | $CH_3$ | $CH_3$ |
| H | H | 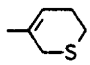 | H | H |
| F | H | 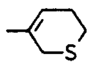 | H | H |
| H | $CH_3$ | 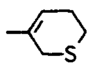 | H | H |
| F | $CH_3$ | 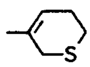 | H | H |
| H | H | 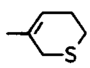 | $CH_3$ | H |
| F | H | 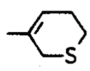 | $CH_3$ | H |
| H | $CH_3$ | 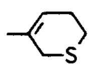 | $CH_3$ | H |
| F | $CH_3$ | 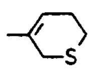 | $CH_3$ | H |
| H | H | 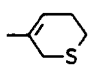 | $CH_3$ | $CH_3$ |
| F | H | 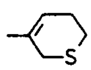 | $CH_3$ | $CH_3$ |
| H | $CH_3$ | 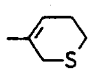 | $CH_3$ | $CH_3$ |
| F | $CH_3$ | 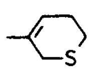 | $CH_3$ | $CH_3$ |
| H | H | 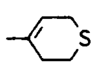 | H | H |

Tabelle III (Fortsetzung)

| $R^1$ | $R^2$ | $R^3$ | $R^4$ | $R^5$ |
|---|---|---|---|---|
| F | H | 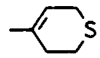 | H | H |
| H | $CH_3$ | 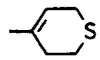 | H | H |
| F | $CH_3$ | 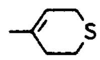 | H | H |
| H | H | 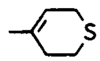 | $CH_3$ | H |
| F | H | 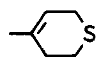 | $CH_3$ | H |
| H | $CH_3$ | 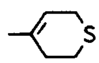 | $CH_3$ | H |
| F | $CH_3$ | 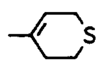 | $CH_3$ | H |
| H | H | 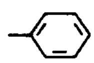 | H | H |
| F | H | 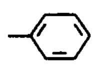 | H | H |
| H | $CH_3$ | 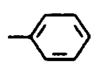 | H | H |
| F | $CH_3$ | 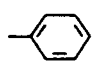 | H | H |
| H | H | 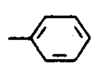 | $CH_3$ | H |
| F | H | 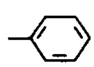 | $CH_3$ | H |
| H | $CH_3$ | 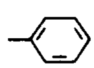 | $CH_3$ | H |
| F | $CH_3$ | 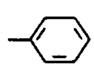 | $CH_3$ | H |
| H | H | 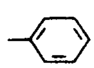 | $CH_3$ | $CH_3$ |
| F | H | 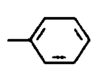 | $CH_3$ | $CH_3$ |

Tabelle III (Fortsetzung)

| $R^1$ | $R^2$ | $R^3$ | $R^4$ | $R^5$ |
|---|---|---|---|---|
| H | $CH_3$ | 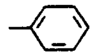 | $CH_3$ | $CH_3$ |
| F | $CH_3$ | 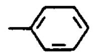 | $CH_3$ | $CH_3$ |
| H | H | 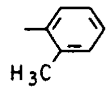 | H | H |
| F | H | 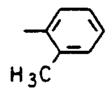 | H | H |
| H | $CH_3$ | 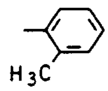 | H | H |
| F | $CH_3$ | 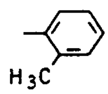 | H | H |
| H | H | 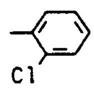 | H | H |
| F | H | 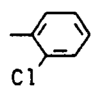 | H | H |
| H | $CH_3$ | 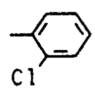 | H | H |
| F | $CH_3$ | 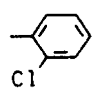 | H | H |
| H | H | 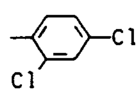 | H | H |
| F | H | 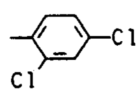 | H | H |
| H | $CH_3$ | 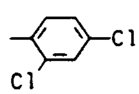 | H | H |
| F | $CH_3$ | 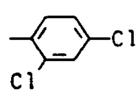 | H | H |

Tabelle IIIa

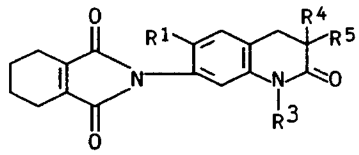

(Y = $-CH_2-CR^4R^5-$, n = 0)

| $R^1$ | $R^4$ | $R^5$ | $R^3$ |
|---|---|---|---|
| H | H | H | H |
| F | H | H | H |
| H | $CH_3$ | H | H |
| F | $CH_3$ | H | H |
| H | H | H | $CH_3$ |
| F | H | H | $CH_3$ |
| H | $CH_3$ | H | $CH_3$ |
| F | $CH_3$ | H | $CH_3$ |
| H | H | H | $CH_2CH_3$ |
| F | H | H | $CH_2CH_3$ |
| H | $CH_3$ | H | $CH_2CH_3$ |
| F | $CH_3$ | H | $CH_2CH_3$ |
| H | H | H | $CH(CH_3)_2$ |
| F | H | H | $CH(CH_3)_2$ |
| H | $CH_3$ | H | $CH(CH_3)_2$ |
| F | $CH_3$ | H | $CH(CH_3)_2$ |
| H | H | H | $CH_2CH=CH_2$ |
| F | H | H | $CH_2CH=CH_2$ |
| H | $CH_3$ | H | $CH_2CH=CH_2$ |
| F | $CH_3$ | H | $CH_2CH=CH_2$ |
| H | H | H | $CH_2CH=CHCH_3$ |
| F | H | H | $CH_2CH=CHCH_3$ |
| H | $CH_3$ | H | $CH_2CH=CHCH_3$ |
| F | $CH_3$ | H | $CH_2CH=CHCH_3$ |
| H | H | H | $CH_2C{\equiv}CH$ |
| F | H | H | $CH_2C{\equiv}CH$ |
| H | $CH_3$ | H | $CH_2C{\equiv}CH$ |
| F | $CH_3$ | H | $CH_2C{\equiv}CH$ |
| H | H | H | $CH_2C{\equiv}CCH_3$ |
| F | H | H | $CH_2C{\equiv}CCH_3$ |
| H | $CH_3$ | H | $CH_2C{\equiv}CCH_3$ |
| F | $CH_3$ | H | $CH_2C{\equiv}CCH_3$ |
| H | H | H | $CH_2OCH_3$ |
| F | H | H | $CH_2OCH_3$ |
| H | $CH_3$ | H | $CH_2OCH_3$ |
| F | $CH_3$ | H | $CH_2OCH_3$ |
| H | H | H | $(CH_2)_2OCH_3$ |
| F | H | H | $(CH_2)_2OCH_3$ |
| H | $CH_3$ | H | $(CH_2)_2OCH_3$ |
| F | $CH_3$ | H | $(CH_2)_2OCH_3$ |
| H | H | H | $CH_2CO_2CH_3$ |
| F | H | H | $CH_2CO_2CH_3$ |
| H | $CH_3$ | H | $CH_2CO_2CH_3$ |
| F | $CH_3$ | H | $CH_2CO_2CH_3$ |
| H | H | H | $CH_2CO_2CH_2CH_3$ |
| F | H | H | $CH_2CO_2CH_2CH_3$ |
| H | $CH_3$ | H | $CH_2CO_2CH_2CH_3$ |
| F | $CH_3$ | H | $CH_2CO_2CH_2CH_3$ |
| H | H | H | $CH(CH_3)CO_2CH_3$ |
| F | H | H | $CH(CH_3)CO_2CH_3$ |
| H | $CH_3$ | H | $CH(CH_3)CO_2CH_3$ |
| F | $CH_3$ | H | $CH(CH_3)CO_2CH_3$ |
| H | H | H | $CH(CH_3)CO_2CH_2CH_3$ |
| F | H | H | $CH(CH_3)CO_2CH_2CH_3$ |
| H | $CH_3$ | H | $CH(CH_3)CO_2CH_2CH_3$ |
| F | $CH_3$ | H | $CH(CH_3)CO_2CH_2CH_3$ |
| H | $CH_3$ | $CH_3$ | H |
| F | $CH_3$ | $CH_3$ | H |

Tabelle IIIa (Fortsetzung)

| $R^1$ | $R^4$ | $R^5$ | $R^3$ |
|---|---|---|---|
| H | $CH_3$ | $CH_3$ | $CH_3$ |
| F | $CH_3$ | $CH_3$ | $CH_3$ |
| H | $CH_3$ | $CH_3$ | $CH_2CH_3$ |
| F | $CH_3$ | $CH_3$ | $CH_2CH_3$ |
| H | $CH_3$ | $CH_3$ | $CH_2CH{=}CH_2$ |
| F | $CH_3$ | $CH_3$ | $CH_2CH{=}CH_2$ |
| H | $CH_3$ | $CH_3$ | $CH_2C{\equiv}CH$ |
| F | $CH_3$ | $CH_3$ | $CH_2C{\equiv}CH$ |
| H | $CH_3$ | $CH_3$ | $(CH_2)_2OCH_3$ |
| F | $CH_3$ | $CH_3$ | $(CH_2)_2OCH_3$ |
| H | $CH_3$ | $CH_3$ | $CH_2CO_2CH_3$ |
| F | $CH_3$ | $CH_3$ | $CH_2CO_2CH_3$ |
| H | $CH_3$ | $CH_3$ | $CH(CH_3)CO_2CH_3$ |
| F | $CH_3$ | $CH_3$ | $CH(CH_3)CO_2CH_3$ |

Tabelle IV

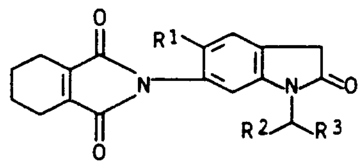

| $R^1$ | $R^2$ | $R^3$ |
|---|---|---|
| H | H | 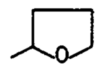 |
| F | H | 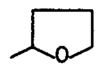 |
| H | $CH_3$ | 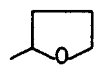 |
| F | $CH_3$ | 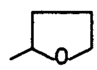 |
| H | H | 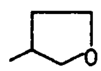 |
| F | H | 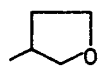 |
| H | $CH_3$ | 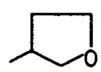 |
| F | $CH_3$ | 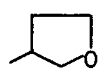 |
| H | H | 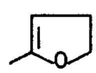 |
| F | H | 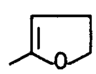 |
| H | $CH_3$ | 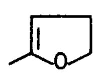 |
| F | $CH_3$ | 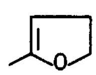 |
| H | H | 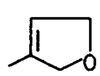 |
| F | H | 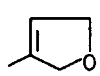 |

Tabelle IV (Fortsetzung)

| $R^1$ | $R^2$ | $R^3$ |
|---|---|---|
| H | $CH_3$ | 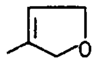 |
| F | $CH_3$ | 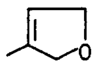 |
| H | H | 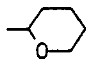 |
| F | H | 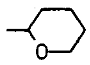 |
| H | $CH_3$ | 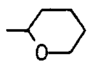 |
| F | $CH_3$ | 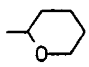 |
| H | H | 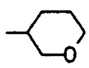 |
| F | H | 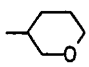 |
| H | $CH_3$ | 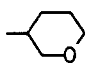 |
| F | $CH_3$ | 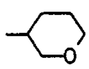 |
| H | H | 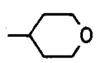 |
| F | H | 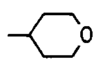 |
| H | $CH_3$ | 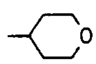 |
| F | $CH_3$ | 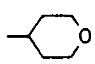 |
| H | H | 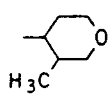 |
| F | H | 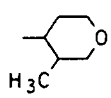 |

Tabelle IV (Fortsetzung)

| $R^1$ | $R^2$ | $R^3$ |
|---|---|---|
| H | $CH_3$ | 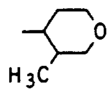 |
| F | $CH_3$ | 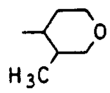 |
| H | H | 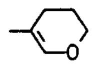 |
| F | H | 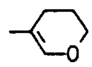 |
| H | $CH_3$ | 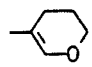 |
| F | $CH_3$ | 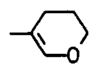 |
| H | H | 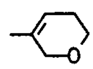 |
| F | H | 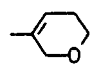 |
| H | $CH_3$ | 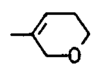 |
| F | $CH_3$ | 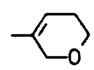 |
| H | H | 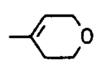 |
| F | H | 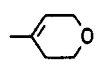 |
| H | $CH_3$ | 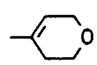 |
| F | $CH_3$ | 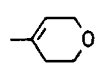 |
| H | H | 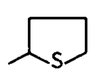 |
| F | H | 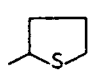 |

Tabelle IV (Fortsetzung)

| $R^1$ | $R^2$ | $R^3$ |
|---|---|---|
| H | $CH_3$ | 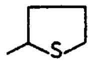 |
| F | $CH_3$ | 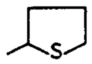 |
| H | H | 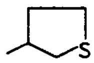 |
| F | H | 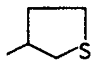 |
| H | $CH_3$ | 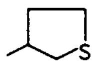 |
| F | $CH_3$ | 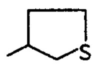 |
| H | H | 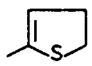 |
| F | H | 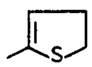 |
| H | $CH_3$ | 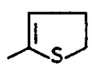 |
| F | $CH_3$ | 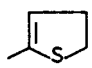 |
| H | H | 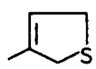 |
| F | H | 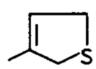 |
| H | $CH_3$ | 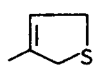 |
| F | $CH_3$ | 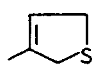 |
| H | H | 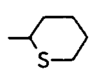 |
| F | H | 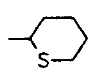 |

Tabelle IV (Fortsetzung)

| $R^1$ | $R^2$ | $R^3$ |
|---|---|---|
| H | $CH_3$ | 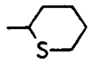 |
| F | $CH_3$ | 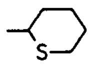 |
| H | H | 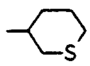 |
| F | H | 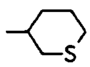 |
| H | $CH_3$ | 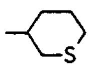 |
| F | $CH_3$ | 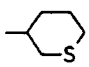 |
| H | H | 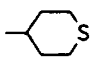 |
| F | H | 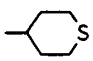 |
| H | $CH_3$ | 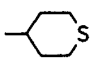 |
| F | $CH_3$ | 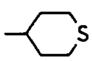 |
| H | H | 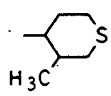 |
| F | H | 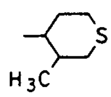 |
| H | $CH_3$ | 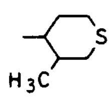 |
| F | $CH_3$ | 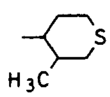 |
| H | H | 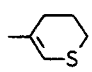 |

Tabelle IV (Fortsetzung)

| $R^1$ | $R^2$ | $R^3$ |
|---|---|---|
| F | H | 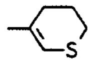 |
| H | $CH_3$ | 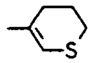 |
| F | $CH_3$ | 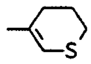 |
| H | H | 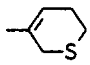 |
| F | H | 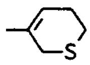 |
| H | $CH_3$ | 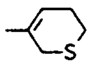 |
| F | $CH_3$ | 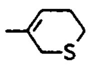 |
| H | H | 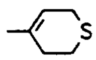 |
| F | H | 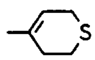 |
| H | $CH_3$ | 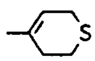 |
| F | $CH_3$ | 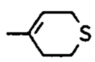 |
| H | H | 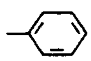 |
| F | H | 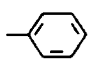 |
| H | $CH_3$ | 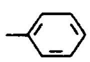 |
| F | $CH_3$ | 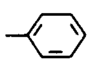 |
| H | H | 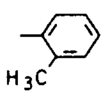 |

Tabelle IV (Fortsetzung)

| $R^1$ | $R^2$ | $R^3$ |
|---|---|---|
| F | H | 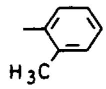 |
| H | $CH_3$ | 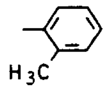 |
| F | $CH_3$ | 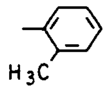 |
| H | H | 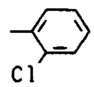 |
| F | H | 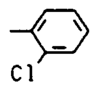 |
| H | $CH_3$ | 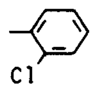 |
| F | $CH_3$ | 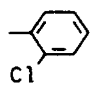 |
| H | H | 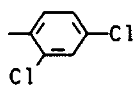 |
| F | H | 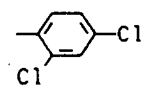 |
| H | $CH_3$ | 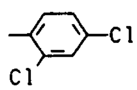 |
| F | $CH_3$ | 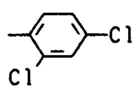 |

Tabelle IVa

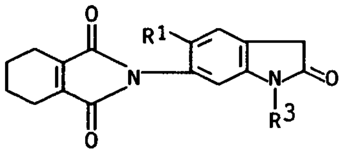

I (Y = $-CH_2-$, n = 0)

| $R^1$ | $R^3$ |
|---|---|
| H | H |
| F | H |
| H | $CH_3$ |
| F | $CH_3$ |
| H | $CH_2CH_3$ |
| F | $CH_2CH_3$ |
| H | $CH(CH_3)_2$ |
| F | $CH(CH_3)_2$ |
| H | $CH_2CH{=}CH_2$ |
| F | $CH_2CH{=}CH_2$ |
| H | $CH_2CH{=}CHCH_3$ |
| F | $CH_2CH{=}CHCH_3$ |
| H | $CH_2C{\equiv}CH$ |
| F | $CH_2C{\equiv}CH$ |
| H | $CH_2C{\equiv}CCH_3$ |
| F | $CH_2C{\equiv}CCH_3$ |
| H | $CH_2OCH_3$ |
| F | $CH_2OCH_3$ |
| H | $(CH_2)_2OCH_3$ |
| F | $(CH_2)_2OCH_3$ |
| H | $CH_2CO_2CH_3$ |
| F | $CH_2CO_2CH_3$ |
| H | $CH_2CO_2CH_2CH_3$ |
| F | $CH_2CO_2CH_2CH_3$ |
| H | $CH(CH_3)CO_2CH_3$ |
| F | $CH(CH_3)CO_2CH_3$ |
| H | $CH(CH_3)CO_2CH_2CH_3$ |
| F | $CH(CH_3)CO_2CH_2CH_3$ |

Tabelle V

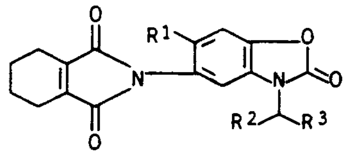

| $R^1$ | $R^2$ | $R^3$ |
|---|---|---|
| H | H | 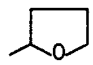 |
| F | H | 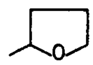 |
| H | $CH_3$ | 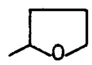 |
| F | $CH_3$ | 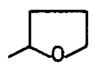 |
| H | H | 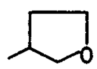 |
| F | H | 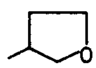 |
| H | $CH_3$ | 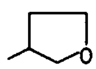 |
| F | $CH_3$ | 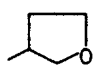 |
| H | H | 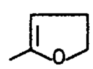 |
| F | H | 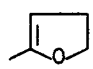 |
| H | $CH_3$ | 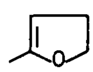 |
| F | $CH_3$ | 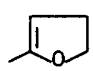 |
| H | H | 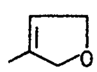 |
| F | H | 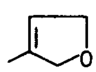 |

Tabelle V (Fortsetzung)

| $R^1$ | $R^2$ | $R^3$ |
|---|---|---|
| H | $CH_3$ | 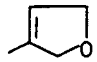 |
| F | $CH_3$ | 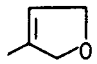 |
| H | H | 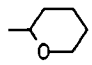 |
| F | H | 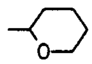 |
| H | $CH_3$ | 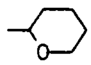 |
| F | $CH_3$ | 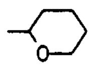 |
| H | H | 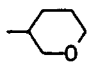 |
| F | H | 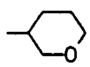 |
| H | $CH_3$ | 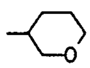 |
| F | $CH_3$ | 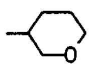 |
| H | H | 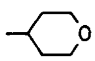 |
| F | H | 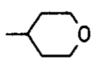 |
| H | $CH_3$ | 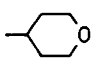 |
| F | $CH_3$ | 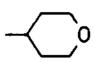 |
| H | H | 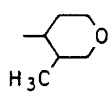 |
| F | H | 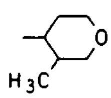 |

Tabelle V (Fortsetzung)

| $R^1$ | $R^2$ | $R^3$ |
|---|---|---|
| H | $CH_3$ | 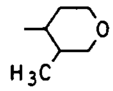 |
| F | $CH_3$ | 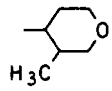 |
| H | H | 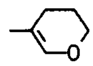 |
| F | H | 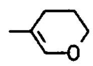 |
| H | $CH_3$ | 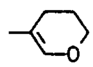 |
| F | $CH_3$ | 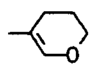 |
| H | H | 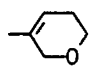 |
| F | H | 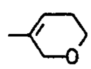 |
| H | $CH_3$ | 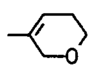 |
| F | $CH_3$ | 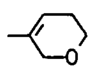 |
| H | H | 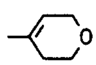 |
| F | H | 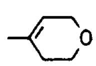 |
| H | $CH_3$ | 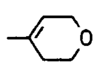 |
| F | $CH_3$ | 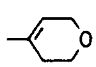 |
| H | H | 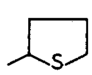 |
| F | H | 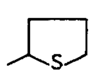 |

Tabelle V (Fortsetzung)

| $R^1$ | $R^2$ | $R^3$ |
|---|---|---|
| H | $CH_3$ | 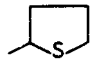 |
| F | $CH_3$ | 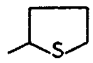 |
| H | H | 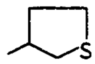 |
| F | H | 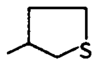 |
| H | $CH_3$ | 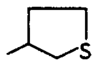 |
| F | $CH_3$ | 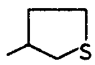 |
| H | H | 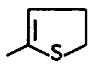 |
| F | H | 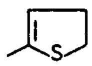 |
| H | $CH_3$ | 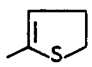 |
| F | $CH_3$ | 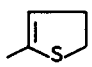 |
| H | H | 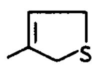 |
| F | H | 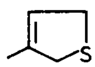 |
| H | $CH_3$ | 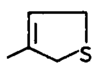 |
| F | $CH_3$ | 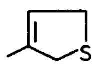 |
| H | H | 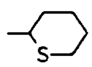 |
| F | H | 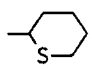 |

Tabelle V (Fortsetzung)

| $R^1$ | $R^2$ | $R^3$ |
|---|---|---|
| H | $CH_3$ | 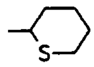 |
| F | $CH_3$ | 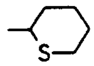 |
| H | H | 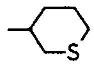 |
| F | H | 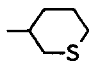 |
| H | $CH_3$ | 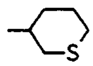 |
| F | $CH_3$ | 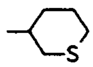 |
| H | H | 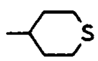 |
| F | H | 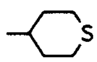 |
| H | $CH_3$ | 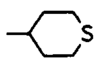 |
| F | $CH_3$ | 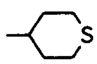 |
| H | H | 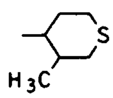 |
| F | H | 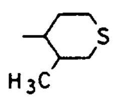 |
| H | $CH_3$ | 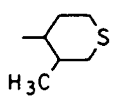 |
| F | $CH_3$ | 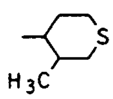 |
| H | H | 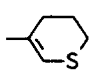 |

Tabelle V (Fortsetzung)

| $R^1$ | $R^2$ | $R^3$ |
|---|---|---|
| F | H | 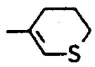 |
| H | $CH_3$ | 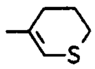 |
| F | $CH_3$ | 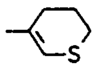 |
| H | H | 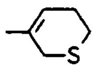 |
| F | H | 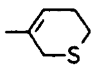 |
| H | $CH_3$ | 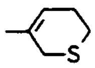 |
| F | $CH_3$ | 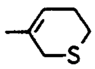 |
| H | H | 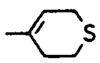 |
| F | H | 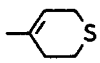 |
| H | $CH_3$ | 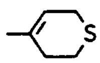 |
| F | $CH_3$ | 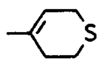 |
| H | H | 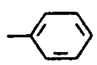 |
| F | H | 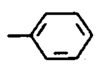 |
| H | $CH_3$ | 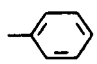 |
| F | $CH_3$ | 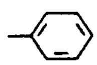 |
| H | H | 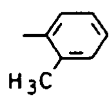 |

Tabelle V (Fortsetzung)

| $R^1$ | $R^2$ | $R^3$ |
|---|---|---|
| F | H | 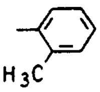 |
| H | $CH_3$ | 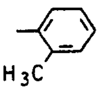 |
| F | $CH_3$ | 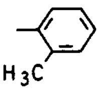 |
| H | H | 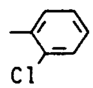 |
| F | H | 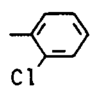 |
| H | $CH_3$ | 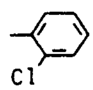 |
| F | $CH_3$ | 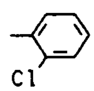 |
| H | H | 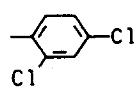 |
| F | H | 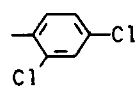 |
| H | $CH_3$ | 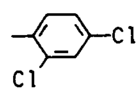 |
| F | $CH_3$ | 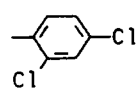 |

Tabelle VI

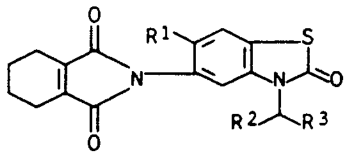

| $R^1$ | $R^2$ | $R^3$ |
|---|---|---|
| H | H | 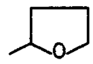 |
| F | H | 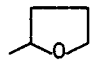 |
| H | $CH_3$ | 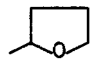 |
| F | $CH_3$ | 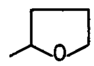 |
| H | H | 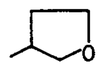 |
| F | H | 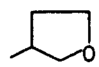 |
| H | $CH_3$ | 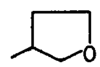 |
| F | $CH_3$ | 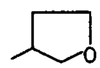 |
| H | H | 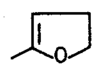 |
| F | H | 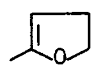 |
| H | $CH_3$ | 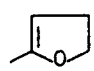 |
| F | $CH_3$ | 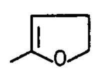 |
| H | H | 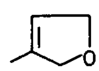 |
| F | H | 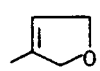 |

Tabelle VI (Fortsetzung)

| $R^1$ | $R^2$ | $R^3$ |
|---|---|---|
| H | $CH_3$ | 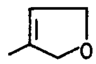 |
| F | $CH_3$ | 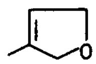 |
| H | H | 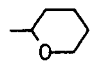 |
| F | H | 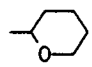 |
| H | $CH_3$ | 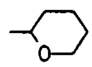 |
| F | $CH_3$ | 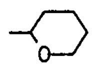 |
| H | H | 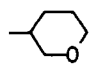 |
| F | H | 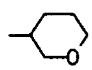 |
| H | $CH_3$ | 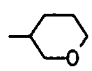 |
| F | $CH_3$ | 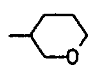 |
| H | H | 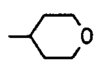 |
| F | H | 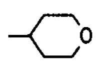 |
| H | $CH_3$ | 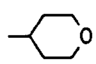 |
| F | $CH_3$ | 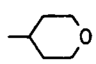 |
| H | H | 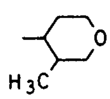 |
| F | H | 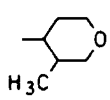 |

Tabelle VI (Fortsetzung)

| $R^1$ | $R^2$ | $R^3$ |
|---|---|---|
| H | $CH_3$ | 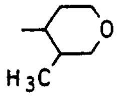 |
| F | $CH_3$ | 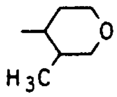 |
| H | H | 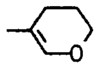 |
| F | H | 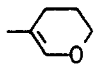 |
| H | $CH_3$ | 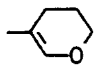 |
| F | $CH_3$ | 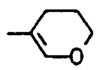 |
| H | H | 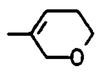 |
| F | H | 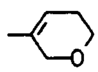 |
| H | $CH_3$ | 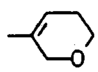 |
| F | $CH_3$ | 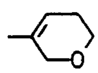 |
| H | H | 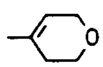 |
| F | H | 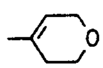 |
| H | $CH_3$ | 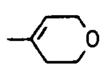 |
| F | $CH_3$ | 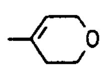 |
| H | H | 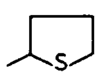 |

Tabelle VI (Fortsetzung)

| $R^1$ | $R^2$ | $R^3$ |
|---|---|---|
| F | H | 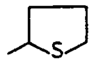 |
| H | $CH_3$ | 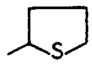 |
| F | $CH_3$ | 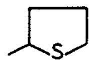 |
| H | H | 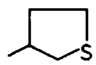 |
| F | H | 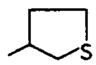 |
| H | $CH_3$ | 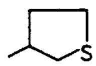 |
| F | $CH_3$ | 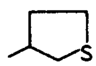 |
| H | H | 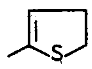 |
| F | H | 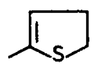 |
| H | $CH_3$ | 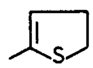 |
| F | $CH_3$ | 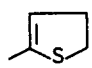 |
| H | H | 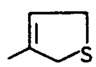 |
| F | H | 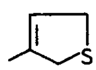 |
| H | $CH_3$ | 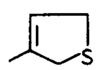 |
| F | $CH_3$ | 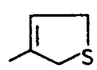 |
| H | H | 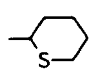 |

Tabelle VI (Fortsetzung)

| $R^1$ | $R^2$ | $R^3$ |
| --- | --- | --- |
| F | H | 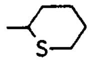 |
| H | $CH_3$ | 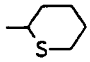 |
| F | $CH_3$ | 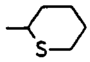 |
| H | H | 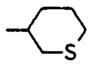 |
| F | H | 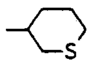 |
| H | $CH_3$ | 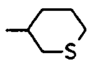 |
| F | $CH_3$ | 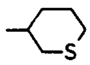 |
| H | H | 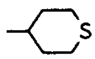 |
| F | H | 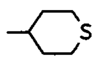 |
| H | $CH_3$ | 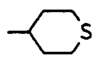 |
| F | $CH_3$ | 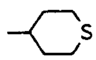 |
| H | H | 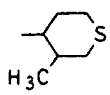 |
| F | H | 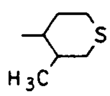 |
| H | $CH_3$ | 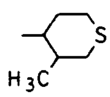 |
| F | $CH_3$ | 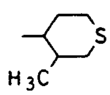 |

Tabelle VI (Fortsetzung)

| $R^1$ | $R^2$ | $R^3$ |
|---|---|---|
| H | H | 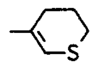 |
| F | H | 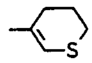 |
| H | $CH_3$ | 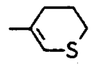 |
| F | $CH_3$ | 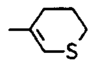 |
| H | H | 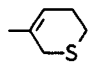 |
| F | H | 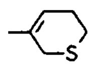 |
| H | $CH_3$ | 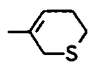 |
| F | $CH_3$ | 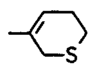 |
| H | H | 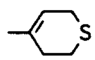 |
| F | H | 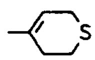 |
| H | $CH_3$ | 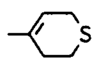 |
| F | $CH_3$ | 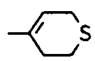 |
| H | H | 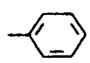 |
| F | H | 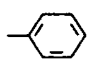 |
| H | $CH_3$ | 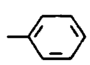 |
| F | $CH_3$ | 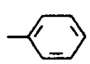 |

Tabelle VI (Fortsetzung)

| $R^1$ | $R^2$ | $R^3$ |
|---|---|---|
| H | H | 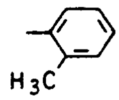 |
| F | H | 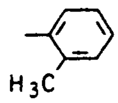 |
| H | $CH_3$ | 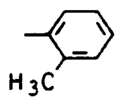 |
| F | $CH_3$ | 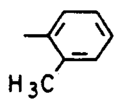 |
| H | H | 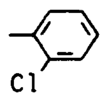 |
| F | H | 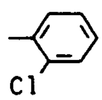 |
| H | $CH_3$ | 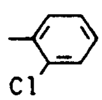 |
| F | $CH_3$ | 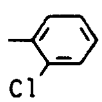 |
| H | H | 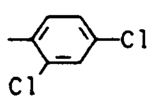 |
| F | H | 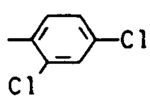 |
| H | $CH_3$ | 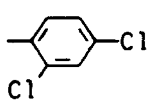 |
| F | $CH_3$ | 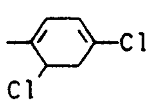 |

Die N-Aryltetrahydrophthalimidverbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten

in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 1.003 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.011 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.035 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3.059 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 4.047 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.023 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.033 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 3.067 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 0,5 kg/ha.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |

| Botanischer Name | Deutscher Name |
|---|---|
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |

| Botanischer Name | Deutscher Name |
|---|---|
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Aryltetrahydrophthalimidverbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Beispiel 1

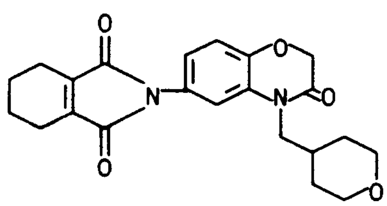

a) 4,9 g (0,03 Mol) 6-Amino-3,4-dihydro-2H-1,4-benzoxazin-3-on werden in 50 ml Dimethylformamid vorgelegt und bei 5°C 0,79 g (0,033 Mol) Natriumhydrid zugegeben und 30 Minuten bei dieser Temperatur gerührt. Danach werden 5,9 g (0,033 Mol) 4-Brommethyl-tetrahydropyran zugegeben, 3 Stunden bei 60°C gerührt, 200 ml Wasser zugegeben, zweimal mit 200 ml Methylenchlorid extrahiert, getrocknet und im Vakuum das Lösungsmittel verdampft. Man erhält 5 g (64 %) 4-(Tetrahydropyran-4-ylmethyl)-6-amino-3,4-dihydro-2H-1,4-benzoxazin-3-on (Öl).

b) 4,5 g (0,017 Mol) des obigen Anilins und 2,7 g (0,018 Mol) Cyclohexen-1,2-dicarbonsäureanhydrid werden in 70 ml Eisessig 3 Stunden bei 70°C gerührt. Nach dem Abkühlen gibt man 100 ml Wasser zu und filtriert ab. Der Niederschlag wird mit Wasser gewaschen und getrocknet. Man erhält 3,5 g (52 %) N-[4-(Tetrahydropyran-4-ylmethyl)-3,4-dihydro-2H-1,4-benzoxazin-3-on-6-yl]-3,4,5,6-tetrahydrophthalimid

(Fp. 187-189 ° C) (Tabelle 1 Nr. 1.033).

Tabelle 1

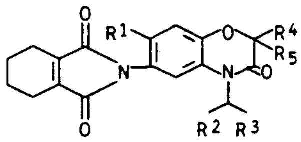

I

| Nr. | $R^1$ | $R^2$ | $R^3$ | $R^4$ | $R^5$ | Fp. [°C] |
|---|---|---|---|---|---|---|
| 1.001 | H | H | 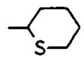 | H | H | |
| 1.002 | F | H | 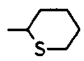 | H | H | |
| 1.003 | H | H | 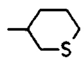 | H | H | 165-166 |
| 1.004 | F | H | 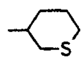 | H | H | 204-206 |
| 1.005 | H | H | 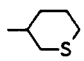 | $CH_3$ | H | |
| 1.006 | F | H | 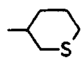 | $CH_3$ | H | |
| 1.007 | H | H | 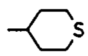 | H | H | |
| 1.008 | F | H | 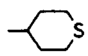 | H | H | |
| 1.009 | H | H | 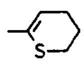 | H | H | |
| 1.010 | F | H | 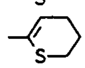 | H | H | |
| 1.011 | H | H | 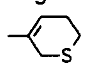 | H | H | 140-142 |
| 1.012 | F | H | 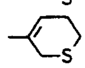 | H | H | |
| 1.013 | H | H | 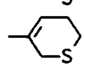 | $CH_3$ | H | |
| 1.014 | F | H | 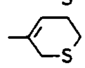 | $CH_3$ | H | |
| 1.015 | H | H | 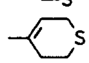 | H | H | |
| 1.016 | F | H | 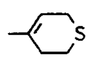 | H | H | |
| 1.017 | H | H | 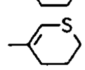 | H | H | |
| 1.018 | F | H | 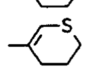 | H | H | |
| 1.019 | H | H | 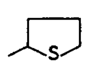 | H | H | |
| 1.020 | F | H | 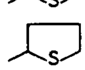 | H | H | |

Tabelle 1 - Fortsetzung -

| Nr. | $R^1$ | $R^2$ | $R^3$ | $R^4$ | $R^5$ | Fp.[°C] |
|---|---|---|---|---|---|---|
| 1.021 | H | H | 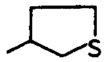 | H | H | |
| 1.022 | F | H | 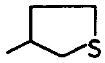 | H | H | |
| 1.023 | H | H | 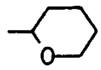 | H | H | 145-147 |
| 1.024 | F | H | 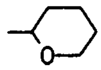 | H | H | |
| 1.025 | H | H | 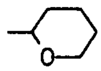 | $CH_3$ | H | |
| 1.026 | F | H | 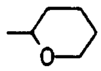 | $CH_3$ | H | |
| 1.027 | H | H | 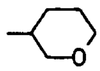 | H | H | 138-140 |
| 1.028 | F | H | 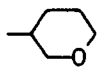 | H | H | 70 (Zers.) |
| 1.029 | H | H | 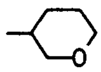 | $CH_3$ | H | 145-147 |
| 1.030 | F | H | 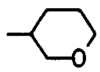 | $CH_3$ | H | |
| 1.031 | H | $CH_3$ | 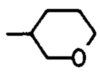 | H | H | |
| 1.032 | F | $CH_3$ | 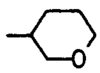 | H | H | |
| 1.033 | H | H | 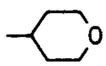 | H | H | 187-189 |
| 1.034 | F | H | 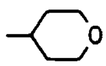 | H | H | |
| 1.035 | H | H | 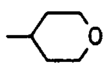 | $CH_3$ | H | 150-152 |
| 1.036 | F | H | 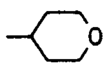 | $CH_3$ | H | |
| 1.037 | H | H | 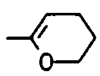 | H | H | |
| 1.038 | F | H | 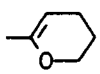 | H | H | |
| 1.039 | H | H | 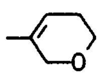 | H | H | |
| 1.040 | F | H | 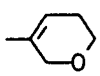 | H | H | 178-179 |

Tabelle 1 - Fortsetzung -

| Nr. | $R^1$ | $R^2$ | $R^3$ | $R^4$ | $R^5$ | Fp. [°C] |
|---|---|---|---|---|---|---|
| 1.041 | H | H | 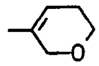 | $CH_3$ | H | |
| 1.042 | F | H | 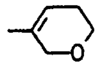 | $CH_3$ | H | |
| 1.043 | H | H | 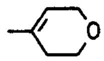 | H | H | |
| 1.044 | F | H | 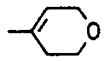 | H | H | |
| 1.045 | H | H | 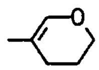 | H | H | |
| 1.046 | F | H | 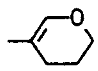 | H | H | |
| 1.047 | H | H | 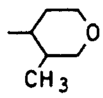 | H | H | |
| 1.048 | F | H | 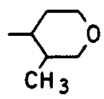 | H | H | |
| 1.049 | H | H | 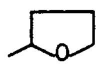 | H | H | 127-129 |
| 1.050 | F | H | 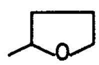 | H | H | |
| 1.051 | H | H | 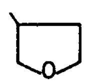 | H | H | |
| 1.052 | F | H | 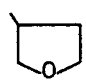 | H | H | |
| 1.053 | H | H | 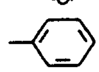 | H | H | 178-180 |
| 1.054 | F | H | 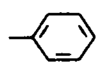 | H | H | |
| 1.055 | H | H | 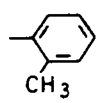 | H | H | 157-168 |
| 1.056 | F | H | 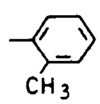 | H | H | |
| 1.057 | H | H | 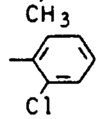 | H | H | 177-178 |
| 1.058 | F | H | 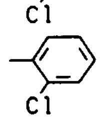 | H | H | |

Tabelle 2

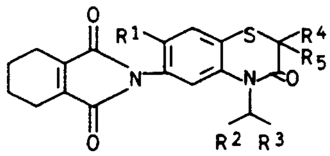

I

| Nr. | $R^1$ | $R^2$ | $R^3$ | $R^4$ | $R^5$ | Fp.[°C] |
|---|---|---|---|---|---|---|
| 2.001 | H | H | 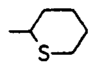 | H | H | |
| 2.002 | F | H | 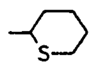 | H | H | |
| 2.003 | H | H | 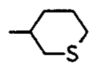 | H | H | |
| 2.004 | F | H | 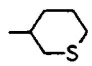 | H | H | |
| 2.005 | H | H | 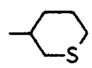 | $CH_3$ | H | |
| 2.006 | F | H | 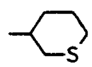 | $CH_3$ | H | |
| 2.007 | H | H | 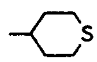 | H | H | |
| 2.008 | F | H | 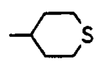 | H | H | |
| 2.009 | H | H | 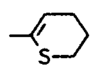 | H | H | |
| 2.010 | F | H | 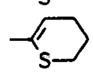 | H | H | |
| 2.011 | H | H | 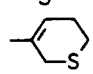 | H | H | |
| 2.012 | F | H | 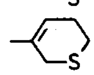 | H | H | |
| 2.013 | H | H | 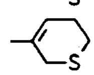 | $CH_3$ | H | |
| 2.014 | F | H | 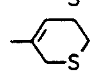 | $CH_3$ | H | |
| 2.015 | H | H | 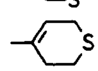 | H | H | |
| 2.016 | F | H | 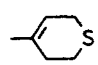 | H | H | |
| 2.017 | H | H | 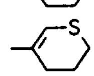 | H | H | |
| 2.018 | F | H | 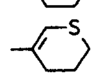 | H | H | |

Tabelle 2 - Fortsetzung -

| Nr. | $R^1$ | $R^2$ | $R^3$ | $R^4$ | $R^5$ | Fp.[°C] |
|---|---|---|---|---|---|---|
| 2.019 | H | H | 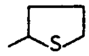 | H | H | |
| 2.020 | F | H | 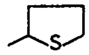 | H | H | |
| 2.021 | H | H | 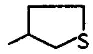 | H | H | |
| 2.022 | F | H | 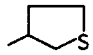 | H | H | |
| 2.023 | H | H | 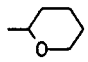 | H | H | |
| 2.024 | F | H | 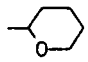 | H | H | |
| 2.025 | H | H | 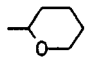 | $CH_3$ | H | |
| 2.026 | F | H | 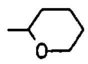 | $CH_3$ | H | |
| 2.027 | H | H | 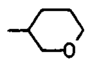 | H | H | |
| 2.028 | F | H | 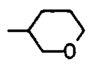 | H | H | |
| 2.029 | H | H | 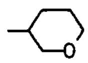 | $CH_3$ | H | |
| 2.030 | F | H | 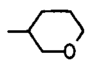 | $CH_3$ | H | |
| 2.031 | H | $CH_3$ | 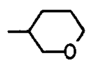 | H | H | |
| 2.032 | F | $CH_3$ | 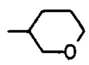 | H | H | |
| 2.033 | H | H | 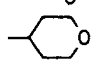 | H | H | |
| 2.034 | F | H | 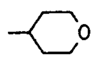 | H | H | |
| 2.035 | H | H | 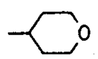 | $CH_3$ | H | |
| 2.036 | F | H | 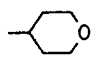 | $CH_3$ | H | |
| 2.037 | H | H | 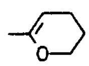 | H | H | |
| 2.038 | F | H | 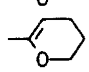 | H | H | |
| 2.039 | H | H | 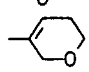 | H | H | |

Tabelle 2 - Fortsetzung -

| Nr. | $R^1$ | $R^2$ | $R^3$ | $R^4$ | $R^5$ | Fp.[°C] |
|---|---|---|---|---|---|---|
| 2.040 | F | H | 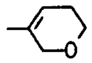 | H | H | |
| 2.041 | H | H | 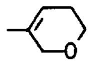 | $CH_3$ | H | |
| 2.042 | F | H | 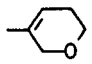 | $CH_3$ | H | |
| 2.043 | H | H | 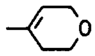 | H | H | |
| 2.044 | F | H | 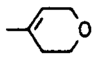 | H | H | |
| 2.045 | H | H | 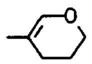 | H | H | |
| 2.046 | F | H | 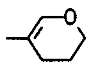 | H | H | |
| 2.047 | H | H | 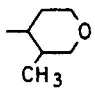 | H | H | |
| 2.048 | F | H | 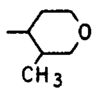 | H | H | |
| 2.049 | H | H | 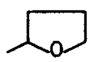 | H | H | |
| 2.050 | F | H | 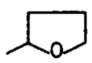 | H | H | |
| 2.051 | H | H | 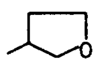 | H | H | |
| 2.052 | F | H | 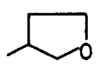 | H | H | |
| 2.053 | H | H | 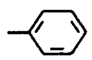 | H | H | |
| 2.054 | F | H | 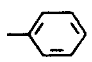 | H | H | |
| 2.055 | H | H | 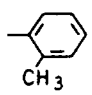 | H | H | |
| 2.056 | F | H | 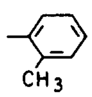 | H | H | |
| 2.057 | H | H | 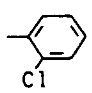 | H | H | |
| 2.058 | F | H | 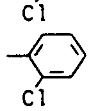 | H | H | |

Tabelle 2a

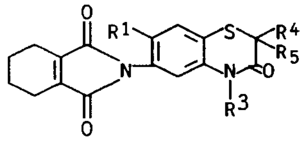

I (Y = $-S-CR^4R^5-$, n = 0)

| Nr. | $R^1$ | $R^3$ | $R^4$ | $R^5$ | Fp.[°C] |
|---|---|---|---|---|---|
| 2.059 | H | H | H | H | 230-231 |
| 2.060 | F | H | H | H | |
| 2.061 | H | $CH_3$ | H | H | |
| 2.062 | F | $CH_3$ | H | H | |
| 2.063 | H | $CH_2CH_3$ | H | H | |
| 2.064 | F | $CH_2CH_3$ | H | H | |
| 2.065 | H | $CH(CH_3)_2$ | H | H | |
| 2.066 | F | $CH(CH_3)_2$ | H | H | |
| 2.067 | H | $CH_2CH{=}CH_2$ | H | H | |
| 2.068 | F | $CH_2CH{=}CH_2$ | H | H | |
| 2.069 | H | $CH_2C{\equiv}CH$ | H | H | 193-194 |
| 2.070 | F | $CH_2C{\equiv}CH$ | H | H | |
| 2.071 | H | $CH_2OCH_3$ | H | H | |
| 2.072 | F | $CH_2OCH_3$ | H | H | |
| 2.073 | H | $CH_2CH_2OCH_3$ | H | H | |
| 2.074 | F | $CH_2CH_2OCH_3$ | H | H | |
| 2.075 | H | $CH_2CO_2CH_3$ | H | H | |
| 2.076 | F | $CH_2CO_2CH_3$ | H | H | |
| 2.077 | H | $CH_2CO_2CH_2CH_3$ | H | H | |
| 2.078 | F | $CH_2CO_2CH_2CH_3$ | H | H | |
| 2.079 | H | $CH(CH_3)CO_2CH_3$ | H | H | |
| 2.080 | F | $CH(CH_3)CO_2CH_3$ | H | H | |
| 2.081 | H | $CH(CH_3)CO_2CH_2CH_3$ | H | H | |
| 2.082 | F | $CH(CH_3)CO_2CH_2CH_3$ | H | H | |
| 2.083 | H | $CH(CH_3)CO_2CH_2CH_3$ | $CH_3$ | H | |
| 2.0084 | F | $CH(CH_3)CO_2CH_2CH_3$ | $CH_3$ | H | |
| 2.085 | H | $CH_2CH{=}CH_2$ | $CH_3$ | H | |
| 2.086 | F | $CH_2CH{=}CH_2$ | $CH_3$ | H | |
| 2.087 | H | $CH_2C{\equiv}CH$ | $CH_3$ | H | |
| 2.088 | F | $CH_2C{\equiv}CH$ | $CH_3$ | H | |

Tabelle 3

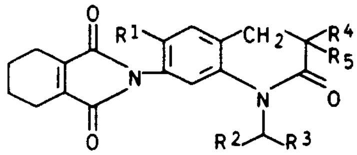

I

| Nr. | $R^1$ | $R^2$ | $R^3$ | $R^4$ | $R^5$ | Fp. [°C] |
|---|---|---|---|---|---|---|
| 3.001 | H | H | 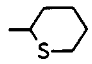 | H | H | |
| 3.002 | F | H | 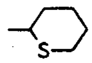 | H | H | |
| 3.003 | H | H | 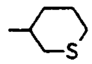 | H | H | |
| 3.004 | F | H | 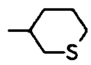 | H | H | |
| 3.005 | H | H | 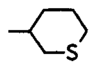 | $CH_3$ | H | |
| 3.006 | F | H | 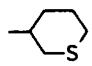 | $CH_3$ | H | |
| 3.007 | H | H | 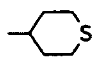 | H | H | |
| 3.008 | F | H | 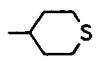 | H | H | |
| 3.009 | H | H | 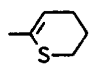 | H | H | |
| 3.010 | F | H | 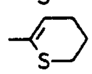 | H | H | |
| 3.011 | H | H | 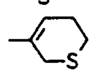 | H | H | 147-149 |
| 3.012 | F | H | 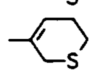 | H | H | |
| 3.013 | H | H | 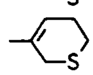 | $CH_3$ | H | |
| 3.014 | F | H | 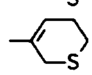 | $CH_3$ | H | |
| 3.015 | H | H | 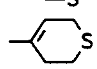 | H | H | |
| 3.016 | F | H | 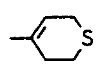 | H | H | |
| 3.017 | H | H | 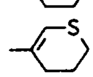 | H | H | |
| 3.018 | F | H | 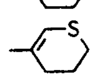 | H | H | |

Tabelle 3 - Fortsetzung -

| Nr. | $R^1$ | $R^2$ | $R^3$ | $R^4$ | $R^5$ | Fp.[°C] |
|---|---|---|---|---|---|---|
| 3.019 | H | H | 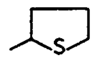 | H | H | |
| 3.020 | F | H | 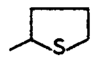 | H | H | |
| 3.021 | H | H | 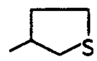 | H | H | |
| 3.022 | F | H | 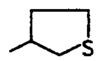 | H | H | |
| 3.023 | H | H | 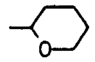 | H | H | |
| 3.024 | F | H | 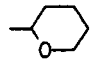 | H | H | |
| 3.025 | H | H | 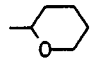 | $CH_3$ | H | |
| 3.026 | F | H | 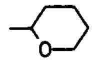 | $CH_3$ | H | |
| 3.027 | H | H | 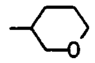 | H | H | 188-190 |
| 3.028 | F | H | 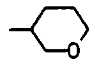 | H | H | |
| 3.029 | H | H | 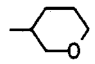 | $CH_3$ | H | |
| 3.030 | F | H | 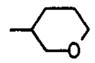 | $CH_3$ | H | |
| 3.031 | H | $CH_3$ | 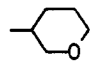 | H | H | |
| 3.032 | F | $CH_3$ | 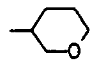 | H | H | |
| 3.033 | H | H | 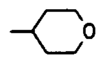 | H | H | |
| 3.034 | F | H | 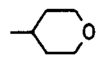 | H | H | |
| 3.035 | H | H | 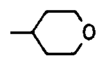 | $CH_3$ | H | |
| 3.036 | F | H | 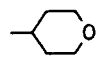 | $CH_3$ | H | |
| 3.037 | H | H | 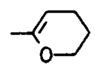 | H | H | |
| 3.038 | F | H | 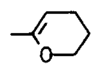 | H | H | |
| 3.039 | H | H | 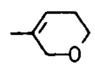 | H | H | |
| 3.040 | F | H | 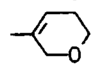 | H | H | |

Tabelle 3 - Fortsetzung -

| Nr. | $R^1$ | $R^2$ | $R^3$ | $R^4$ | $R^5$ | Fp.[°C] |
|---|---|---|---|---|---|---|
| 3.041 | H | H | 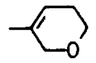 | $CH_3$ | H | |
| 3.042 | F | H | 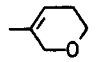 | $CH_3$ | H | |
| 3.043 | H | H | 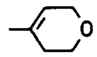 | H | H | |
| 3.044 | F | H | 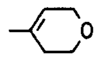 | H | H | |
| 3.045 | H | H | 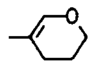 | H | H | |
| 3.046 | F | H | 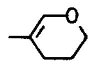 | H | H | |
| 3.047 | H | H | 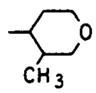 | H | H | |
| 3.048 | F | H | 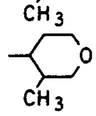 | H | H | |
| 3.049 | H | H | 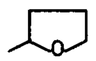 | H | H | |
| 3.050 | F | H | 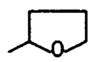 | H | H | |
| 3.051 | H | H | 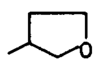 | H | H | |
| 3.052 | F | H | 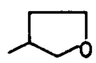 | H | H | |
| 3.053 | H | H | 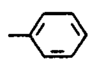 | H | H | |
| 3.054 | F | H | 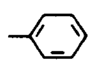 | H | H | |
| 3.055 | H | H | 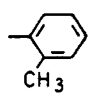 | H | H | 173-175 |
| 3.056 | F | H | 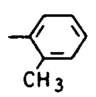 | H | H | |
| 3.057 | H | H | 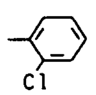 | H | H | |
| 3.058 | F | H | 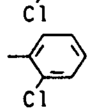 | H | H | |

Tabelle 3a

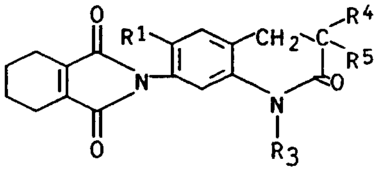

I (Y = $-CH_2-CR^4R^5-$, n = 0)

| Nr. | $R^1$ | $R_3$ | $R^4$ | $R^5$ | Fp. [°C] |
|---|---|---|---|---|---|
| 3.059 | H | H | H | H | 237-238 |
| 3.060 | F | H | H | H | |
| 3.061 | H | $CH_3$ | H | H | 232-234 |
| 3.062 | F | $CH_3$ | H | H | |
| 3.063 | H | $CH_2CH_3$ | H | H | |
| 3.064 | F | $CH_2CH_3$ | H | H | |
| 3.065 | H | $CH(CH_3)_2$ | H | H | |
| 3.066 | F | $CH(CH_3)_2$ | H | H | |
| 3.067 | H | $CH_2CH{=}CH_2$ | H | H | 168-170 |
| 3.068 | F | $CH_2CH{=}CH_2$ | H | H | |
| 3.069 | H | $CH_2C{\equiv}CH$ | H | H | 196-198 |
| 3.070 | F | $CH_2C{\equiv}CH$ | H | H | |
| 3.071 | H | $CH_2OCH_3$ | H | H | |
| 3.072 | F | $CH_2OCH_3$ | H | H | |
| 3.073 | H | $CH_2CH_2OCH_3$ | H | H | |
| 3.074 | F | $CH_2CH_2OCH_3$ | H | H | |
| 3.075 | H | $CH_2CO_2CH_3$ | H | H | |
| 3.076 | F | $CH_2CO_2CH_3$ | H | H | |
| 3.077 | H | $CH_2CO_2CH_2CH_3$ | H | H | |
| 3.078 | F | $CH_2CO_2CH_2CH_3$ | H | H | |
| 3.079 | H | $CH(CH_3)CO_2CH_3$ | H | H | |
| 3.080 | F | $CH(CH_3)CO_2CH_3$ | H | H | |
| 3.081 | H | $CH(CH_3)CO_2CH_2CH_3$ | H | H | |
| 3.082 | F | $CH(CH_3)CO_2CH_2CH_3$ | H | H | |
| 3.083 | H | $CH(CH_3)CO_2CH_2CH_3$ | $CH_3$ | H | |
| 3.084 | F | $CH(CH_3)CO_2CH_2CH_3$ | $CH_3$ | H | |
| 3.085 | H | $CH_2CH{=}CH_2$ | $CH_3$ | H | |
| 3.086 | F | $CH_2CH{=}CH_2$ | $CH_3$ | H | |
| 3.087 | H | $CH_2C{\equiv}CH$ | $CH_3$ | H | |
| 3.088 | F | $CH_2C{\equiv}CH$ | $CH_3$ | H | |

Tabelle 4

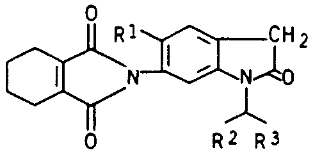

I

| Nr. | $R^1$ | $R^2$ | $R^3$ | Fp.[°C] |
|---|---|---|---|---|
| 4.001 | H | H | 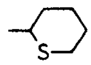 | |
| 4.002 | F | H | 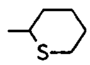 | |
| 4.003 | H | H | 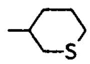 | |
| 4.004 | F | H | 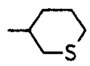 | |
| 4.005 | H | H | 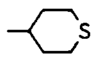 | |
| 4.006 | F | H | 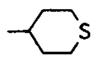 | |
| 4.007 | H | H | 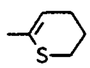 | |
| 4.008 | F | H | 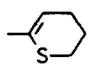 | |
| 4.009 | H | H | 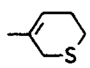 | |
| 4.010 | F | H | 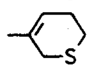 | |
| 4.011 | H | H | 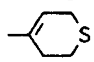 | |
| 4.012 | F | H | 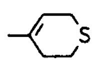 | |
| 4.013 | H | H | 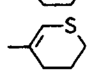 | |
| 4.014 | F | H | 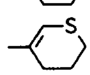 | |
| 4.015 | H | H | 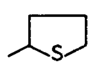 | |
| 4.016 | H | H | 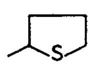 | |
| 4.017 | H | H | 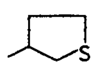 | |
| 4.018 | F | H | 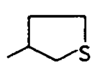 | |

Tabelle 4 - Fortsetzung -

| Nr. | $R^1$ | $R^2$ | $R^3$ | Fp.[°C] |
|---|---|---|---|---|
| 4.019 | H | H | 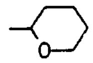 | |
| 4.020 | F | H | 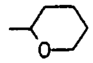 | |
| 4.021 | H | H | 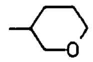 | 150-152 |
| 4.022 | F | H | 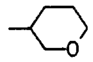 | |
| 4.023 | H | $CH_3$ | 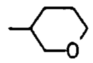 | |
| 4.024 | F | $CH_3$ | 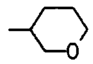 | |
| 4.025 | H | H | 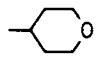 | |
| 4.026 | F | H | 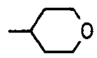 | |
| 4.027 | H | H | 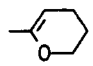 | |
| 4.028 | F | H | 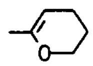 | |
| 4.029 | H | H | 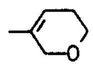 | |
| 4.030 | F | H | 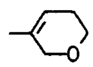 | |
| 4.031 | H | H | 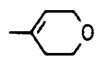 | |
| 4.032 | F | H | 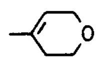 | |
| 4.033 | H | H | 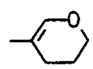 | |
| 4.034 | F | H | 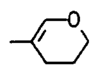 | |
| 4.035 | H | H | 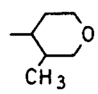 | |
| 4.036 | F | H | 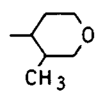 | |
| 4.037 | H | H | 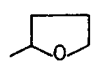 | |
| 4.038 | F | H | 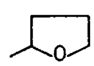 | |
| 4.039 | H | H | 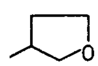 | |
| 4.040 | F | H | 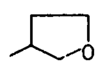 | |

Tabelle 4 - Fortsetzung -

| Nr. | $R^1$ | $R^2$ | $R^3$ | Fp.[°C] |
|---|---|---|---|---|
| 4.041 | H | H | 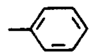 | |
| 4.042 | F | H | 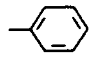 | |
| 4.043 | H | H | 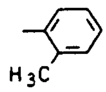 | |
| 4.044 | F | H | 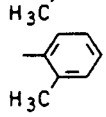 | |
| 4.045 | H | H | 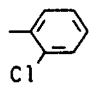 | |
| 4.046 | F | H | 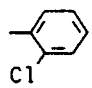 | |

Tabelle 4a

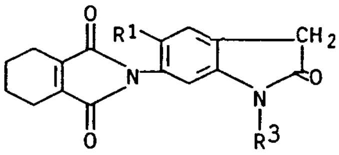

I (Y = -CÓH$_2$-, n = 0)

| Nr. | R1 | R3 | Fp.[°C] |
|---|---|---|---|
| 4.047 | H | H | 140-142 |
| 4.048 | F | H | |
| 4.049 | H | $CH_3$ | 205-207 |
| 4.050 | F | $CH_3$ | |
| 4.051 | H | $CH_2CH_3$ | |
| 4.052 | F | $CH_2CH_3$ | |
| 4.053 | H | $CH(CH_3)_2$ | |
| 4.054 | F | $CH(CH_3)_2$ | |
| 4.055 | H | $CH_2CH=CH_2$ | |
| 4.056 | F | $CH_2CH=CH_2$ | |
| 4.057 | H | $CH_2C\equiv CH$ | |
| 4.058 | F | $CH_2C\equiv CH$ | |
| 4.059 | H | $CH_2OCH_3$ | |
| 4.060 | F | $CH_2OCH_3$ | |
| 4.061 | H | $CH_2CH_2OCH_3$ | |
| 4.062 | F | $CH_2CH_2OCH_3$ | |
| 4.063 | H | $CH_2CO_2CH_3$ | |
| 4.064 | F | $CH_2CO_2CH_3$ | |
| 4.065 | H | $CH_2CO_2CH_2CH_3$ | |
| 4.066 | F | $CH_2CO_2CH_2CH_3$ | |
| 4.067 | H | $CH(CH_3)CO_2CH_3$ | |
| 4.068 | F | $CH(CH_3)CO_2CH_3$ | |
| 4.069 | H | $CH(CH_3)CO_2CH_2CH_3$ | |
| 4.070 | F | $CH(CH_3)CO_2CH_2CH_3$ | |

Tabelle 5

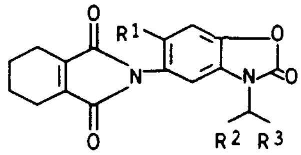

I

| Nr. | $R^1$ | $R^2$ | $R^3$ | Fp.[°C] |
|---|---|---|---|---|
| 5.001 | H | H | 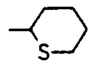 | |
| 5.002 | F | H | 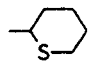 | |
| 5.003 | H | H | 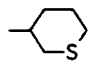 | |
| 5.004 | F | H | 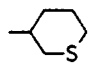 | |
| 5.005 | H | H | 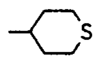 | |
| 5.006 | F | H | 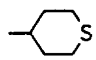 | |
| 5.007 | H | H | 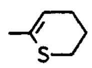 | |
| 5.008 | F | H | 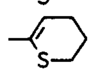 | |
| 5.009 | H | H | 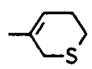 | 147-151 |
| 5.010 | F | H | 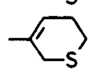 | |
| 5.011 | H | H | 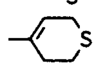 | |
| 5.012 | F | H | 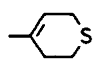 | |
| 5.013 | H | H | 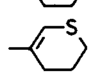 | |
| 5.014 | F | H | 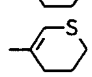 | |
| 5.015 | H | H | 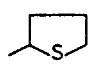 | |
| 5.016 | F | H | 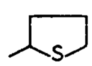 | |
| 5.017 | H | H | 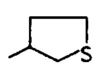 | |
| 5.018 | F | H | 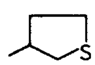 | |

Tabelle 5 - Fortsetzung -

| Nr. | $R^1$ | $R^2$ | $R^3$ | Fp.[°C] |
|---|---|---|---|---|
| 5.019 | H | H | 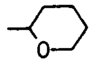 | 133-135 |
| 5.020 | F | H | 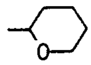 | |
| 5.021 | H | H | 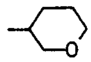 | 167-169 |
| 5.022 | F | H | 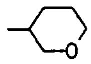 | |
| 5.023 | H | $CH_3$ | 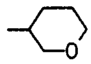 | |
| 5.024 | F | $CH_3$ | 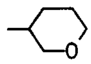 | |
| 5.025 | H | H | 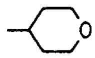 | |
| 5.026 | F | H | 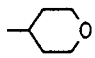 | |
| 5.027 | H | H | 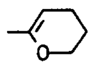 | |
| 5.028 | F | H | 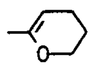 | |
| 5.029 | H | H | 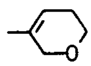 | |
| 5.030 | F | H | 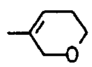 | |
| 5.031 | H | H | 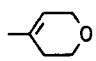 | |
| 5.032 | F | H | 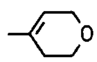 | |
| 5.033 | H | H | 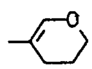 | |
| 5.034 | F | H | 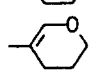 | |
| 5.035 | H | H | 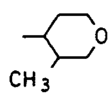 | |
| 5.036 | F | H | 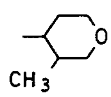 | |
| 5.037 | H | H | 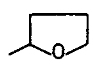 | |
| 5.038 | F | H | 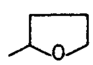 | |
| 5.039 | H | H | 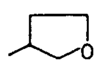 | |
| 5.040 | F | H | 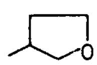 | |

Tabelle 5 - Fortsetzung -

| Nr. | $R^1$ | $R^2$ | $R^3$ | Fp.[°C] |
|---|---|---|---|---|
| 5.041 | H | H | 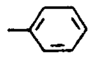 | 206-207 |
| 5.042 | F | H | 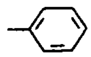 | |
| 5.043 | H | H | 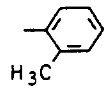 | |
| 5.044 | F | H | 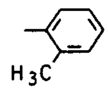 | |
| 5.045 | H | H | 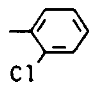 | |
| 5.046 | F | H | 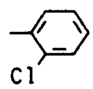 | |

Tabelle 6

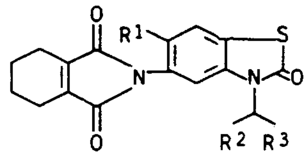

I

| Nr. | $R^1$ | $R^2$ | $R^3$ | Fp.[°C] |
|---|---|---|---|---|
| 6.001 | H | H | 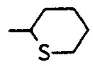 | |
| 6.002 | F | H | 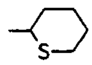 | |
| 6.003 | H | H | 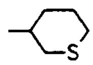 | |
| 6.004 | F | H | 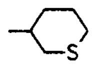 | |
| 6.005 | H | H | 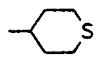 | |
| 6.006 | F | H | 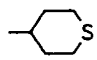 | |
| 6.007 | H | H | 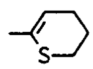 | |
| 6.008 | F | H | 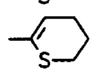 | |
| 6.009 | H | H | 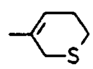 | |
| 6.010 | F | H | 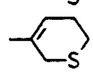 | |
| 6.011 | H | H | 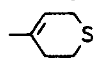 | |
| 6.012 | F | H | 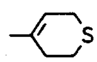 | |
| 6.013 | H | H | 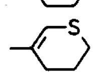 | |
| 6.014 | F | H | 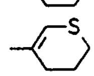 | |
| 6.015 | H | H | 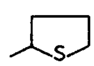 | |
| 6.016 | F | H | 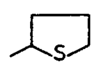 | |
| 6.017 | H | H | 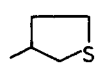 | |
| 6.018 | F | H | 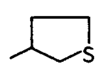 | |

Tabelle 6 - Fortsetzung -

| Nr. | $R^1$ | $R^2$ | $R^3$ | Fp.[°C] |
|---|---|---|---|---|
| 6.019 | H | H | 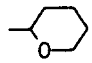 | |
| 6.020 | F | H | 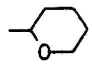 | |
| 6.021 | H | H | 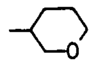 | |
| 6.022 | F | H | 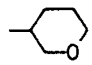 | |
| 6.023 | H | $CH_3$ | 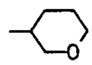 | |
| 6.024 | F | $CH_3$ | 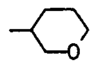 | |
| 6.025 | H | H | 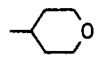 | |
| 6.026 | F | H | 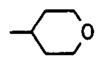 | |
| 6.027 | H | H | 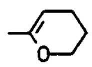 | |
| 6.028 | F | H | 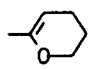 | |
| 6.029 | H | H | 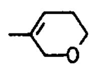 | |
| 6.030 | F | H | 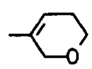 | 187-189 |
| 6.031 | H | H | 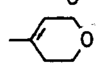 | |
| 6.032 | F | H | 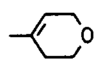 | |
| 6.033 | H | H | 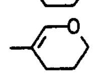 | |
| 6.034 | F | H | 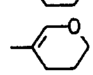 | |
| 6.035 | H | H | 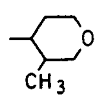 | |
| 6.036 | F | H | 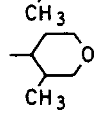 | |
| 6.037 | H | H | 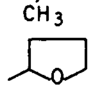 | |
| 6.038 | F | H | 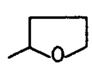 | |
| 6.039 | H | H | 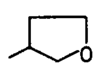 | |

Tabelle 6 - Fortsetzung -

| Nr. | $R^1$ | $R^2$ | $R^3$ | Fp.[°C] |
|---|---|---|---|---|
| 6.040 | F | H | 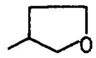 | |
| 6.041 | H | H | 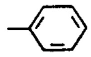 | |
| 6.042 | F | H | 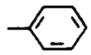 | |
| 6.043 | H | H | 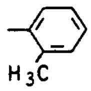 | |
| 6.044 | F | H | 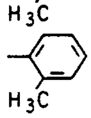 | |
| 6.045 | H | H | 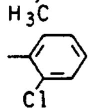 | |
| 6.046 | F | H | 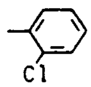 | |

Anwendungsbeispiele

Die Wirkung der N-Aryltetrahydrophthalimidverbindungen der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 und 0,125 kg/ha a.S.

Die Versuchsgefäße werden im Gewächshaus aufgestellt, wobei für wärmeliebende Arten wärmere Bereiche (20 bis 35 ° C) und für solche gemäßigter Klimate 10 bis 20 ° C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Amaranthus retroflexus | Zurückgekrümmter Fuchsschwanz |
| Galium aparine | Klettenlaubkraut |
| Stellaria media | Vogelsternmiere |
| Veronica spp. | Ehrenpreisarten |
| Oryza sativa | Reis |
| Solanum nigrum | Schwarzer Nachtschatten |
| Stellaria media | Vogelmiere |
| Triticum aestivum | Winterweizen |

Mit 0,06 bzw. 0,125 kg/ha a.S. (aktive Substanz) im Nachauflaufverfahren eingesetzt, lassen sich mit Bsp. 1.027 bzw. Bsp. 1.029 breitblättrige unerwünschte Pflanzen sehr gut bekämpfen, und zwar mit

gleichzeitiger Verträglichkeit für Reis bzw. für Weizen. Bsp. 3.067 bekämpft bei 0,06 kg/ha a.S. unerwünschte breitblättrige Pflanzen im Nachauflaufverfahren ohne Reis als Beispielkultur nennenswert zu schädigen.

## Patentansprüche

**Patentansprüche für folgende Vertragsstaaten : BE, CH, DE, FR, GB, IT, LI, NL**

**1.** N-Aryltetrahydrophthalimidverbindungen der allgemeinen Formel I,

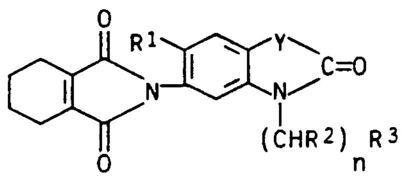

I

in der die Substituenten und Indices folgende Bedeutung haben:

n die Zahl 0 oder 1

$R^1$ Wasserstoff oder Halogen

Y -O-, -S-, $-CH_2-$
$-S-CR^4R^5-$,
$-O-CR^4R^5-$,
$-CH_2-CR^4R^5-$,

wobei $R^4$ und $R^5$ unabhängig voneinander Wasserstoff oder $C_1$-$C_3$-Alkyl bedeuten,

$R^2$ Wasserstoff oder $C_1$-$C_3$-Alkyl

$R^3$ einen gesättigten oder einfach ungesättigten Fünf- oder Sechsring mit einem Sauerstoff- oder Schwefelatom im Ring, der durch $C_1$-$C_3$-Alkyl ein- bis dreifach substituiert sein kann oder unsubstituiertes oder ein- bis dreifach durch $C_1$-$C_2$-Alkyl, Halogen, Methoxy, Nitro und/oder Cyano substituiertes Phenyl und
sofern n = 0 ist und Y die Bedeutung $-CH_2-$, $-CH_2-CR^4R^5-$ oder $-S-CR^4R^5-$ hat,
$R^3$ zusätzlich Wasserstoff, $C_1$-$C_5$-Alkyl, $C_3$-$C_4$-Alkenyl, $C_3$-$C_4$-Alkinyl, $C_1$-$C_4$-Haloalkyl, $C_3$-$C_4$-Haloalkenyl, $C_1$-$C_2$-Alkoxy-($C_1$-$C_2$)-alkyl, $C_1$-$C_2$-Alkoxy-($C_1$-$C_2$)-alkoxy-($C_1$-$C_2$)-alkyl oder durch $C_1$-$C_6$-Alkoxycarbonyl substituiertes $C_1$-$C_3$-Alkyl.

**2.** Verfahren zur Herstellung von Verbindungen der Formel I gemäß Anspruch 1, dadurch gekennzeichnet, daß man ein Amin der Formel II

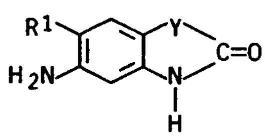

II

mit einer entsprechenden N-alkylierenden Verbindung $Z-(CHR^2)_nR^3$, worin Z eine übliche nucleophile Abgangsgruppe bedeutet, umsetzt und das erhaltene Amin der Formel III

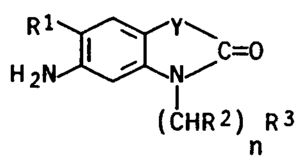

III

mit Tetrahydrophthalsäureanhydrid zu I kondensiert.

3. Verfahren zur Herstellung von N-Aryltetrahydrophthalimidverbindungen der Formel I gemäß Anspruch 1, dadurch gekennzeichnet, daß man ein Amin der Formel II gemäß Anspruch 2, zunächst mit Tetrahydrophthalsäureanhydrid zum N-(Phenyl)tetrahydrophthalimid IV

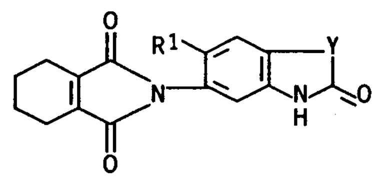

umsetzt und dann mit einer entsprechenden N-alkylierenden Verbindung $Z\text{-}(CHR^2)_nR^3$ gemäß Anspruch 2 zu I N-alkyliert.

4. Verwendung der N-Aryltetrahydrophthalimidverbindungen der Formel I gemäß Anspruch 1 als Herbizide.

5. Herbizide Mittel, enthaltend eine N-Aryltetrahydrophthalimidverbindung der Formel I gemäß Anspruch 1 und inerte Zusatzstoffe.

6. Herbizide Mittel gemäß Anspruch 5, enthaltend eine N-Aryltetrahydrophthalimidverbindung der Formel I und weitere wirksame Bestandteile.

7. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge einer N-Aryltetrahydrophthalimidverbindung I gemäß Anspruch 1 behandelt.

**Patentansprüche für folgenden Vertragsstaat : ES**

1. Verfahren zur Herstellung von N-Aryltetrahydrophthalimidverbindungen der allgemeinen Formel I,

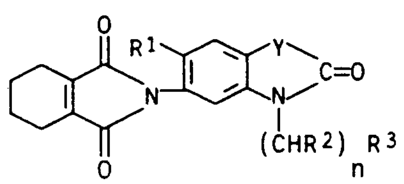

I

in der die Substituenten und Indices folgende Bedeutung haben:

n die Zahl 0 oder 1
$R^1$ Wasserstoff oder Halogen
Y -O-, -S-, $-CH_2-$
$-S-CR^4R^5-$,
$-O-CR^4R^5-$,
$-CH_2-CR^4R^5-$,

wobei $R^4$ und $R^5$ unabhängig voneinander Wasserstoff oder $C_1$-$C_3$-Alkyl bedeuten,

$R^2$ Wasserstoff oder $C_1$-$C_3$-Alkyl
$R^3$ einen gesättigten oder einfach ungesättigten Fünf- oder Sechsring mit einem Sauerstoff- oder Schwefelatom im Ring, der durch $C_1$-$C_3$-Alkyl ein- bis dreifach substituiert sein kann oder unsubstituiertes oder ein- bis dreifach durch $C_1$-$C_2$-Alkyl, Halogen, Methoxy, Nitro und/oder Cyano substituiertes Phenyl und
sofern n = 0 ist und Y die Bedeutung $-CH_2-$, $-CH_2-CR^4R^5-$ oder $-S-CR^4R^5-$ hat,
$R^3$ zusätzlich Wasserstoff, $C_1$-$C_5$-Alkyl, $C_3$-$C_4$-Alkenyl, $C_3$-$C_4$-Alkinyl, $C_1$-$C_4$-Haloalkyl, $C_3$-$C_4$-Haloalkenyl, $C_1$-$C_2$-Alkoxy-($C_1$-$C_2$)-alkyl, $C_1$-$C_2$-Alkoxy-($C_1$-$C_2$)-alkoxy-($C_1$-$C_2$)-alkyl oder durch $C_1$-$C_6$-Alkoxycarbonyl substituiertes $C_1$-$C_3$-Alkyl,

dadurch gekennzeichnt, daß man ein Amin der Formel II

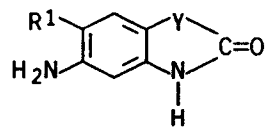

II

mit einer entsprechenden N-alkylierenden Verbindung Z-$(CHR^2)_nR^3$, worin Z eine übliche nucleophile Abgangsgruppe bedeutet, umsetzt und das erhaltene Amin der Formel III

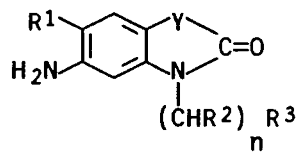

III

mit Tetrahydrophthalsäureanhydrid zu I kondensiert.

2. Verfahren zur Herstellung von N-Aryltetrahydrophthalimidverbindungen der Formel I gemäß Anspruch 1, dadurch gekennzeichnet, daß man ein Amin der Formel II zunächst mit Tetrahydrophthalsäureanhydrid zum N-(Phenyl)tetrahydrophthalimid IV

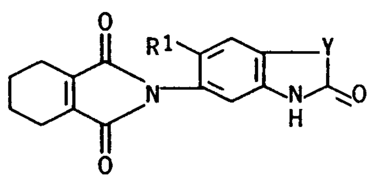

umsetzt und dann mit einer entsprechenden N-alkylierenden Verbindung Z-$(CHR^2)_nR^3$ gemäß Anspruch 1 zu I N-alkyliert.

3. Verwendung der N-Aryltetrahydrophthalimidverbindungen der Formel I gemäß Anspruch 1 als Herbizide.

4. Herbizide Mittel, enthaltend eine N-Aryltetrahydrophthalimidverbindung der Formel I gemäß Anspruch 1 und inerte Zusatzstoffe.

5. Herbizide Mittel gemäß Anspruch 4, enthaltend eine N-Aryltetrahydrophthalimidverbindung der Formel I und weitere wirksame Bestandteile.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge einer N-Aryltetrahydrophthalimidverbindung I gemäß Anspruch 1 behandelt.

**Claims**
**Claims for the following Contracting States : BE, CH, DE, FR, GB, IT, LI, NL**

1. An N-aryltetrahydrophthalimide compound of the formula I

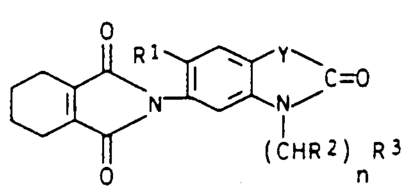

I

where the substituents and indices have the following meanings:
n is 0 or 1, $R^1$ is hydrogen or halogen, Y is -O-, -S-, $-CH_2-$, $-S-CR^4R^5-$, $-O-CR^4R^5-$ or $-CH_2-CR^4R^5-$, $R^4$ and $R^5$ independently of one another being hydrogen or $C_1$-$C_3$-alkyl, $R^2$ is hydrogen or $C_1$-$C_3$-alkyl, $R^3$ is a saturated or monounsaturated 5-membered or 6-membered ring which has an oxygen or sulfur atom in the ring and may be monosubstituted to trisubstituted by $C_1$-$C_3$-alkyl, or $R^3$ is phenyl which is unsubstituted or monosubstituted to trisubstituted by $C_1$- or $C_2$-alkyl, halogen, methoxy, nitro and/or cyano, and, if n is 0 and Y is $-CH_2-$, $-CH_2-CR^4R^5-$ or $-S-CR^4R^5-$, $R^3$ may furthermore be hydrogen, $C_1$-$C_5$-alkyl, $C_3$- or $C_4$-alkenyl, $C_3$- or $C_4$-alkynyl, $C_1$-$C_4$-haloalkyl, $C_3$- or $C_4$-haloalkenyl, $C_1$- or $C_2$-alkoxy-$C_1$- or $C_2$-alkyl, $C_1$- or $C_2$-alkoxy-$C_1$- or $C_2$-alkoxy-$C_1$- or $C_2$-alkyl or $C_1$-$C_6$-alkoxycarbonyl-substituted $C_1$-$C_3$-alkyl.

2. A process for the preparation of a compound of the formula I as claimed in claim 1, wherein an amine of the formula II

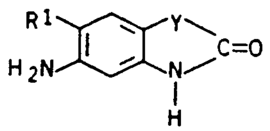

II

is reacted with a corresponding N-alkylating compound $Z-(CHR^2)_nR^3$, where Z is a conventional nucleophilic leading group, and the resulting amine of the formula III

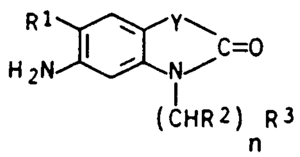

III

is condensed with tetrahydrophthalic anhydride to give I.

3. A process for the preparation of an N-aryltetrahydrophthalimide compound of the formula I as claimed in claim 1, wherein an amine of the formula II as claimed in claim 2 is first reacted with tetrahydrophthalic anhydride to give the N-(phenyl)tetrahydrophthalimide IV

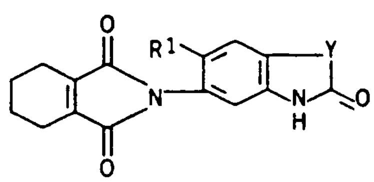

and is then N-alkylated with a corresponding N-alkylating compound $Z-(CHR^2)_nR^3$ as claimed in claim 2 to give I.

4. Use of an N-aryltetrahydrophthalimide compound of the formula I as claimed in claim 1 as a herbicide.

5. A herbicide containing an N-aryltetrahydrophthalimide compound of the formula I as claimed in claim 1 and inert additives.

6. A herbicide as claimed in claim 2, containing an N-aryltetrahydrophthalimide compound of the formula I and further active constituents.

7. A method for controlling undesirable plant growth, wherein the undesirable plants and/or their habitat are treated with a herbicidally effective amount of an N-aryltetrahydrophthalimide compound I, as

claimed in claim 1.

**Claims for the following Contracting State : ES**

**1.** A process for the preparation of an N-aryltetrahydrophthalimide compound of the formula I

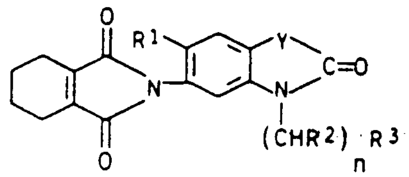

I

where the substituents and indices have the following meanings: n is 0 or 1, $R^1$ is hydrogen or halogen, Y is -O-, -S-, $-CH_2-$, $-S-CR^4R^5-$, $-O-CR^4R^5-$ or $-CH_2-CR^4R^5-$, $R^4$ and $R^5$ independently of one another being hydrogen or $C_1$-$C_3$-alkyl, $R^2$ is hydrogen or $C_1$-$C_3$-alkyl, $R^3$ is a saturated or monounsaturated 5-membered or 6-membered ring which has an oxygen or sulfur atom in the ring and may be monosubstituted to trisubstituted by $C_1$-$C_3$-alkyl, or $R^3$ is phenyl which is unsubstituted or monosubstituted to trisubstituted by $C_1$- or $C_2$-alkyl, halogen, methoxy, nitro and/or cyano, and, if n is 0 and Y is $-CH_2-$, $-CH_2-CR^4R^5-$ or $-S-CR^4R^5-$, $R^3$ may furthermore be hydrogen, $C_1$-$C_5$-alkyl, $C_3$- or $C_4$-alkenyl, $C_3$- or $C_4$-alkynyl, $C_1$-$C_4$-haloalkyl, $C_3$- or $C_4$-haloalkenyl, $C_1$- or $C_2$-alkoxy-$C_1$- or $C_2$-alkyl, $C_1$- or $C_2$-alkoxy-$C_1$- or $C_2$-alkoxy-$C_1$- or $C_2$-alkyl or $C_1$-$C_6$-alkoxycarbonyl-substituted $C_1$-$C_3$-alkyl,
wherein an amine of the formula II

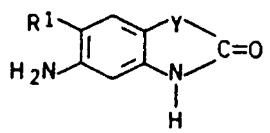

II

is reacted with a corresponding N-alkylating compound $Z-(CHR^2)_nR^3$, where Z is a conventional nucleophilic leaving group, and the resulting amine of the formula III

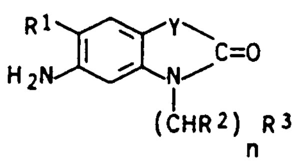

III

is condensed with tetrahydrophthalic anhydride to give I.

**2.** A process for the preparation of an N-aryltetrahyrophthalimide compound of the formula I as claimed in claim 1, wherein an amine of the formula II is first reacted with tetrahydrophthalic anhydride to give the N-(phenyl)tetrahydrophthalimide IV

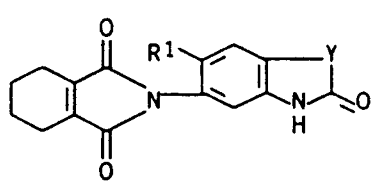

and is then N-alkylated with a corresponding N-alkylating compound $Z-(CHR^2)_nR^3$ as claimed in claim 1 to give I.

**3.** Use of an N-aryltetrahydrophthalimide compound of the formula I as claimed in claim 1 as a herbicide.

**4.** A herbicide containing an N-aryltetrahydrophthalimide compound of the formula I as claimed in claim 1 and inert additives.

**5.** A herbicide as claimed in claim 4, containing an N-aryltetrahydrophthalimide compound of the formula I and further active constituents.

**6.** A method for controlling undesirable plant growth, wherein the undesirable plants and/or their habitat are treated with a herbicidally effective amount of an N-aryltetrahydrophthalimide compound I, as claimed in claim 1.

**Revendications**

**Revendications pour les Etats contractants suivants : BE, CH, DE, FR, GB, IT, LI, NL**

**1.** N-aryltétrahydrophtalimides qui répondent à la formule générale I

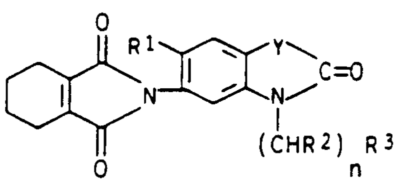

I

dans laquelle les substituants et les indices possèdent les significations suivantes :

- n représente 0 ou 1
- $R^1$ représente l'hydrogène ou un atome d'halogène
- Y représente
  - -O-, -S-, $-CH_2-$
  - $-S-CR^4R^5-$,
  - $-O-CR^4R^5-$,
  - $-CH_2-CR^4R^5-$,

où $R^4$ et $R^5$ représentent chacun, indépendamment l'un de l'autre, un atome d'hydrogène ou un radical alkyle en $C_1$-$C_3$,

- $R^2$ représente l'hydrogène ou un radical alkyle en $C_1$-$C_3$,
- $R^3$ représente un noyau pentagonal ou hexagonal, saturé ou mono-insaturé, avec un atome d'oxygène ou de soufre dans le cycle, qui peut être mono- à trisubstitué par des radicaux alkyle en $C_1$ à $C_3$, ou qui peut ne pas être substitué, ou un radical phényle mono à trisubstitué par des atomes d'halogènes ou des radicaux alkyle en $C_1$-$C_2$, méthoxy, nitro et/ou cyano et

  pour autant que n = 0 et que Y représente $-CH_2-$, $-CH_2-CR^4R^5-$ ou $-S-CR^4R^5-$,

  $R^3$ peut complémentairement représenter un atome d'hydrogène, un radical alkyle en $C_1$-$C_5$, alcényle en $C_3$-$C_4$, alcynyle en $C_3$-$C_4$, haloalkyle en $C_1$-$C_4$, haloalcényle en $C_3$-$C_4$, alcoxy-($C_1$-$C_2$)alkyle($C_1$-$C_2$), alcoxy($C_1$-$C_2$)-alcoxy($C_1$-$C_2$)-alkyle($C_1$-$C_2$), ou alkyle en $C_1$-$C_3$ à substitution alcoxy($C_1$-$C_6$)carbonylique.

**2.** Procédé de préparation de composés de la formule I suivant la revendication 1, caractérisé en ce que l'on fait réagir une amine de la formule II

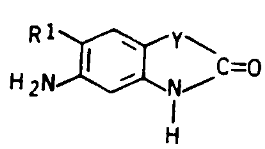

II

avec un composé N-alkylant correspondant de la formule $Z-(CHR^2)_nR^3$ dans laquelle Z représente un

groupe labile ou sortant, nucléophile, usuel et on condense l'amine obtenue de la formule III

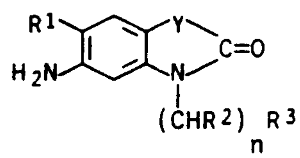

III

avec l'anhydride tétrahydrophtalique en le composé I.

3. Procédé de préparation de N-aryltétrahydrophtalimides de la formule I suivant la revendication 1, caractérisé en ce que l'on convertit d'abord une amine de la formule II selon la revendication 2 avec l'anhydride tétrahydrophtalique en N-(phényl)tétrahydrophtalimide IV

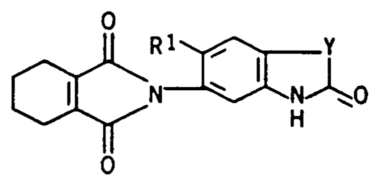

IV

et on le N-alkyle ensuite avec un composé N-alkylant correspondant Z-$(CHR^2)_nR^3$ selon la revendication 2 de manière à obtenir le composé I.

4. Utilisation des N-aryltétrahydrophtalimides de la formule I selon la revendication 1 à titre d'herbicides.

5. Agents ou compositions herbicides qui contiennent un N-aryltétrahydrophtalimide de la formule I selon la revendication 1 et des additifs inertes.

6. Agents ou compositions herbicides selon la revendication 5, qui contiennent un N-aryltétrahydrophtalimide de la formule I et d'autres composants actifs.

7. Procédé de lutte contre la croissance de plantes indésirables, caractérisé en ce que l'on traite les plantes indésirables et/ou leur biotope par une proportion efficace du point de vue herbicide d'un N-aryltétrahydrophtalimide I selon la revendication 1.

**Revendications pour l'Etat contractant suivant : ES**

1. Procédé de préparation de N-aryltétrahydrophtalimides qui répondent à la formule générale I

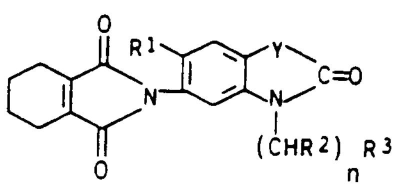

I

dans laquelle les substituants et les indices possèdent les significations suivantes :

n représente 0 ou 1

$R^1$ représente l'hydrogène ou un atome d'halogène

Y représente

-O-, -S-, $-CH_2-$

$-S-CR^4R^5-$,

$-O-CR^4R^5-$,

$-CH_2-CR^4R^5-$,

où $R^4$ et $R^5$ représentent chacun, indépendamment l'un de l'autre, un atome d'hydrogène ou un radical alkyle en $C_1$-$C_3$,

$R^2$ représente l'hydrogène ou un radical alkyle en $C_1$-$C_3$,

$R^3$ représente un noyau pentagonal ou hexagonal, saturé ou mono-insaturé, avec un atome d'oxygène ou de soufre dans le cycle, qui peut être mono- à trisubstitué par des radicaux alkyle en $C_1$ à $C_3$, ou qui peut ne pas être substitué, ou un radical phényle mono à trisubstitué par des atomes d'halogènes ou des radicaux alkyle en $C_1$-$C_2$, méthoxy, nitro et/ou cyano et

pour autant que n = 0 et que Y représente $-CH_2-$, $-CH_2-CR^4R^5-$ ou $-S-CR^4R^5-$,

$R^3$ peut complémentairement représenter un atome d'hydrogène, un radical alkyle en $C_1$-$C_5$, alcényle en $C_3$-$C_4$, alcynyle en $C_3$-$C_4$, haloalkyle en $C_1$-$C_4$, haloalcényle en $C_3$-$C_4$, alcoxy-($C_1$-$C_2$)alkyle($C_1$-$C_2$), alcoxy($C_1$-$C_2$)-alcoxy($C_1$-$C_2$)-alkyle($C_1$-$C_2$), ou alkyle en $C_1$-$C_3$ à substitution alcoxy($C_1$-$C_6$)carbonylique,

caractérisé en ce que l'on fait réagir une amine de la formule II

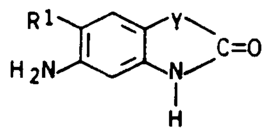

II

avec un composé N-alkylant correspondant de la formule $Z-(CHR^2)_nR^3$ dans laquelle Z représente un groupe labile ou sortant, nucléophile, usuel et on condense l'amine obtenue de la formule III

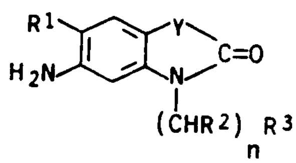

III

avec l'anhydride tétrahydrophtalique en le composé I.

2. Procédé de préparation de N-aryltétrahydrophtalimides de la formule I selon la revendication 1, caractérisé en ce que l'on convertit d'abord une amine de la formule II avec l'anhydride tétrahydrophtalique en N-(phényl)tétrahydrophtalimide IV

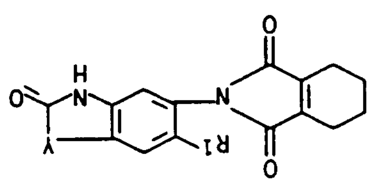

et on la N)-alkyle ensuite avec un composé N-alkylant correspondant $Z-(CHR^2)_nR^3$ selon la revendication 1 de façon à obtenir le composé I.

3. Utilisation des N-aryltétrahydrophtalimides de la formule I selon la revendication 1 à titre d'herbicides.

4. Agents ou compositions herbicides qui contiennent un N-aryltétrahydrophtalimide de la formule I selon la revendication 1 et des additifs inertes.

5. Agents ou compositions herbicides selon la revendication 4, qui contiennent un N-aryltétrahydrophtalimide de la formule I et d'autres composants actifs.

**6.** Procédé de lutte contre la croissance de plantes indésirables, caractérisé en ce que l'on traite les plantes indésirables et/ou leur biotope par une proportion efficace du point de vue herbicide d'un N-aryltétrahydrophtalimide I selon la revendication 1.